# EUROPEAN PATENT APPLICATION

(11) **EP 2 062 971 A1**
(43) Date of publication of application: **27.05.2009**
(21) Application number: 07805909.4
(22) Date of filing: 09.08.2007
(51) Int. Cl.: C12N 7/00, A61K 35/14, A61K 35/28, A61K 35/76, A61P 35/00, C12N 15/09

(54) **CHIMERIC ADENOVIRUS, METHOD FOR PRODUCING THE SAME AND PHARMACEUTICAL USING THE SAME**

(30) Priority: 22.08.2006 JP 2006225392
(71) Applicant: Chiba-ken, Chiba-shi, Chiba 260-8667 (JP)
(72) Inventor: TAGAWA, Masatoshi, Chiba-shi Chiba 260-0801 (JP); KAWAMURA, Kiyoko, Chiba-shi Chiba 267-0057 (JP); TAKENOBU, Hisanori, Chiba-shi Chiba 260-0801 (JP)
(74) Representative: Hallybone, Huw George
(86) International application number: PCT/JP2007/065578
(87) International publication number: WO 2008/023572

(57) **Abstract**

The invention relates to novel chimeric adenoviruses comprising a type 5-modified chimeric adenovirus, in which a fiber knob domain in an adenovirus type 5 is replaced by an adenovirus type 35 fiber knob domain and any extraneous transcriptional regulatory domain controlling expression of E1A and E1B genes is introduced into a region from which an adenovirus type 5 E1A transcriptional regulatory domain has been removed. The chimeric adenoviruses are cytolytic or oncolytic chimeric adenoviruses and can be utilized as, for example, medicaments having high cytotoxic activity against intractable tumors such as malignant mesothelioma.

SEQUENCE LISTING

<110> Chiba-ken

<120> Chimeric adenovirus, method for producing the same and pharmaceutical
using the same

<130> P052713EP

<150> JP 2006-225392
<151> 2006-08-22

<160> 3

<170> PatentIn version 3.1

<210> 1

<211> 32407

<212> DNA

<213> adenovirus

<400> 1

cgtaactata acggtcctaa ggtagcgaaa attatttaaa tatctatgtc gggtgcggag 60

aaagaggtaa tgaaatggca tcgactcgaa gatctgggcg tggttaaggg tgggaaagaa 120

tatataaggt gggggtctta tgtagttttg tatctgtttt gcagcagccg ccgccgccat 180

gagcaccaac tcgtttgatg gaagcattgt gagctcatat ttgacaacgc gcatgccccc 240

atgggccggg gtgcgtcaga atgtgatggg ctccagcatt gatggtcgcc ccgtcctgcc 300

cgcaaactct actaccttga cctacgagac cgtgtctgga acgccgttgg agactgcagc 360

ctccgccgcc gcttcagccg ctgcagccac cgcccgcggg attgtgactg actttgcttt 420

cctgagcccg cttgcaagca gtgcagcttc ccgttcatcc gcccgcgatg acaagttgac 480

ggctcttttg gcacaattgg attctttgac ccgggaactt aatgtcgttt ctcagcagct 540

gttggatctg cgccagcagg tttctgccct gaaggcttcc tcccctccca atgcggttta 600

aaacataaat aaaaaaccag actctgtttg gatttggatc aagcaagtgt cttgctgtct 660

ttatttaggg gttttgcgcg cgcggtaggc ccgggaccag cggtctcggt cgttgagggt 720

cctgtgtatt ttttccagga cgtggtaaag gtgactctgg atgttcagat acatgggcat 780

aagcccgtct ctggggtgga ggtagcacca ctgcagagct tcatgctgcg gggtggtgtt 840

gtagatgatc cagtcgtagc aggagcgctg ggcgtggtgc ctaaaaatgt ctttcagtag 900

caagctgatt gccaggggca ggcccttggt gtaagtgttt acaaagcggt taagctggga 960

tgggtgcata cgtggggata tgagatgcat cttggactgt atttttaggt tggctatgtt 1020

cccagccata tccctccggg gattcatgtt gtgcagaacc accagcacag tgtatccggt 1080

gcacttggga aatttgtcat gtagcttaga aggaaatgcg tggaagaact tggagacgcc 1140

cttgtgacct ccaagatttt ccatgcattc gtccataatg atggcaatgg gcccacgggc 1200

ggcggcctgg gcgaagatat ttctgggatc actaacgtca tagttgtgtt ccaggatgag 1260

atcgtcatag gccattttta caaagcgcgg gcggagggtg ccagactgcg gtataatggt 1320

tccatccggc ccaggggcgt agttaccctc acagatttgc atttcccacg ctttgagttc 1380

agatgggggg atcatgtcta cctgcggggc gatgaagaaa acggtttccg gggtagggga 1440

gatcagctgg gaagaaagca ggttcctgag cagctgcgac ttaccgcagc cggtgggccc 1500

gtaaatcaca cctattaccg gctgcaactg gtagttaaga gagctgcagc tgccgtcatc 1560

cctgagcagg ggggccactt cgttaagcat gtccctgact cgcatgtttt ccctgaccaa 1620

atccgccaga aggcgctcgc cgcccagcga tagcagttct tgcaaggaag caaagttttt 1680

caacggtttg agaccgtccg ccgtaggcat gcttttgagc gtttgaccaa gcagttccag 1740

gcggtcccac agctcggtca cctgctctac ggcatctcga tccagcatat ctcctcgttt 1800

cgcgggttgg ggcggctttc gctgtacggc agtagtcggt gctcgtccag acgggccagg 1860

gtcatgtctt tccacgggcg cagggtcctc gtcagcgtag tctgggtcac ggtgaagggg 1920

tgcgctccgg gctgcgcgct ggccagggtg cgcttgaggc tggtcctgct ggtgctgaag 1980

cgctgccggt cttcgccctg cgcgtcggcc aggtagcatt tgaccatggt gtcatagtcc 2040

agcccctccg cggcgtggcc cttggcgcgc agcttgccct tggaggaggc gccgcacgag 2100

gggcagtgca gacttttgag ggcgtagagc ttgggcgcga gaaataccga ttccggggag 2160

taggcatccg cgccgcaggc cccgcagacg gtctcgcatt ccacgagcca ggtgagctct 2220

ggccgttcgg ggtcaaaaac caggtttccc ccatgctttt tgatgcgttt cttacctctg 2280

gtttccatga gccggtgtcc acgctcggtg acgaaaaggc tgtccgtgtc cccgtataca 2340

gacttgagag gcctgtcctc gagcggtgtt ccgcggtcct cctcgtatag aaactcggac 2400

cactctgaga caaaggctcg cgtccaggcc agcacgaagg aggctaagtg ggaggggtag 2460

cggtcgttgt ccactagggg gtccactcgc tccagggtgt gaagacacat gtcgccctct 2520

tcggcatcaa ggaaggtgat tggtttgtag gtgtaggcca cgtgaccggg tgttcctgaa 2580

ggggggctat aaaagggggt gggggcgcgt tcgtcctcac tctcttccgc atcgctgtct 2640

gcgagggcca gctgttgggg tgagtactcc ctctgaaaag cgggcatgac ttctgcgcta 2700

agattgtcag tttccaaaaa cgaggaggat ttgatattca cctggcccgc ggtgatgcct 2760

ttgagggtgg ccgcatccat ctggtcagaa aagacaatct ttttgttgtc aagcttggtg 2820

gcaaacgacc cgtagagggc gttggacagc aacttggcga tggagcgcag ggtttggttt 2880

ttgtcgcgat cggcgcgctc cttggccgcg atgtttagct gcacgtattc gcgcgcaacg 2940

caccgccatt cgggaaagac ggtggtgcgc tcgtcgggca ccaggtgcac gcgccaaccg 3000

cggttgtgca gggtgacaag gtcaacgctg gtggctacct ctccgcgtag gcgctcgttg 3060

gtccagcaga ggcggccgcc cttgcgcgag cagaatggcg gtagggggtc tagctgcgtc 3120

tcgtccgggg ggtctgcgtc cacggtaaag accccgggca gcaggcgcgc gtcgaagtag 3180

tctatcttgc atccttgcaa gtctagcgcc tgctgccatg cgcgggcggc aagcgcgcgc 3240

tcgtatgggt tgagtggggg accccatggc atggggtggg tgagcgcgga ggcgtacatg 3300

ccgcaaatgt cgtaaacgta gaggggctct ctgagtattc caagatatgt agggtagcat 3360

cttccaccgc ggatgctggc gcgcacgtaa tcgtatagtt cgtgcgaggg agcgaggagg 3420

tcgggaccga ggttgctacg ggcgggctgc tctgctcgga agactatctg cctgaagatg 3480

gcatgtgagt tggatgatat ggttggacgc tggaagacgt tgaagctggc gtctgtgaga 3540

cctaccgcgt cacgcacgaa ggaggcgtag gagtcgcgca gcttgttgac cagctcggcg 3600

gtgacctgca cgtctagggc gcagtagtcc agggtttcct tgatgatgtc atacttatcc 3660

tgtccctttt ttttccacag ctcgcggttg aggacaaact cttcgcggtc tttccagtac 3720

tcttggatcg gaaacccgtc ggcctccgaa cggtaagagc ctagcatgta gaactggttg 3780

acggcctggt aggcgcagca tcccttttct acgggtagcg cgtatgcctg cgcggccttc 3840

cggagcgagg tgtgggtgag cgcaaaggtg tccctgacca tgactttgag gtactggtat 3900

ttgaagtcag tgtcgtcgca tccgccctgc tcccagagca aaaagtccgt gcgctttttg 3960

gaacgcggat ttggcagggc gaaggtgaca tcgttgaaga gtatctttcc cgcgcgaggc 4020

ataaagttgc gtgtgatgcg gaagggtccc ggcacctcgg aacggttgtt aattacctgg 4080

gcggcgagca cgatctcgtc aaagccgttg atgttgtggc ccacaatgta aagttccaag 4140

aagcgcggga tgcccttgat ggaaggcaat tttttaagtt cctcgtaggt gagctcttca 4200

ggggagctga gcccgtgctc tgaaagggcc cagtctgcaa gatgagggtt ggaagcgacg 4260

aatgagctcc acaggtcacg ggccattagc atttgcaggt ggtcgcgaaa ggtcctaaac 4320

tggcgaccta tggccatttt ttctggggtg atgcagtaga aggtaagcgg gtcttgttcc 4380

cagcggtccc atccaaggtt cgcggctagg tctcgcgcgg cagtcactag aggctcatct 4440

ccgccgaact tcatgaccag catgaagggc acgagctgct tcccaaaggc ccccatccaa 4500

gtataggtct ctacatcgta ggtgacaaag agacgctcgg tgcgaggatg cgagccgatc 4560

gggaagaact ggatctcccg ccaccaattg gaggagtggc tattgatgtg gtgaaagtag 4620

aagtccctgc gacgggccga acactcgtgc tggcttttgt aaaaacgtgc gcagtactgg 4680

cagcggtgca cgggctgtac atcctgcacg aggttgacct gacgaccgcg cacaaggaag 4740

cagagtggga atttgagccc ctcgcctggc gggtttggct ggtggtcttc tacttcggct 4800

gcttgtcctt gaccgtctgg ctgctcgagg ggagttacgg tggatcggac caccacgccg 4860

cgcgagccca aagtccagat gtccgcgcgc ggcggtcgga gcttgatgac aacatcgcgc 4920

agatgggagc tgtccatggt ctggagctcc cgcggcgtca ggtcaggcgg gagctcctgc 4980

aggtttacct cgcatagacg ggtcagggcg cgggctagat ccaggtgata cctaatttcc 5040

aggggctggt tggtggcggc gtcgatggct tgcaagaggc cgcatccccg cggcgcgact 5100

acggtaccgc gcggcgggcg gtgggccgcg ggggtgtcct tggatgatgc atctaaaagc 5160

ggtgacgcgg gcgagccccc ggaggtaggg ggggctccgg acccgccggg agagggggca 5220
ggggcacgtc ggcgccgcgc gcgggcagga gctggtgctg cgcgcgtagg ttgctggcga 5280
acgcgacgac gcggcggttg atctcctgaa tctggcgcct ctgcgtgaag acgacgggcc 5340
cggtgagctt gaacctgaaa gagagttcga cagaatcaat ttcggtgtcg ttgacggcgg 5400
cctggcgcaa aatctcctgc acgtctcctg agttgtcttg ataggcgatc tcggccatga 5460
actgctcgat ctcttcctcc tggagatctc cgcgtccggc tcgctccacg gtggcggcga 5520
ggtcgttgga aatgcgggcc atgagctgcg agaaggcgtt gaggcctccc tcgttccaga 5580
cgcggctgta gaccacgccc ccttcggcat cgcgggcgcg catgaccacc tgcgcgagat 5640
tgagctccac gtgccgggcg aagacggcgt agtttcgcag gcgctgaaag aggtagttga 5700
gggtggtggc ggtgtgttct gccacgaaga agtacataac ccagcgtcgc aacgtggatt 5760
cgttgatatc ccccaaggcc tcaaggcgct ccatggcctc gtagaagtcc acggcgaagt 5820
tgaaaaactg ggagttgcgc gccgacacgg ttaactcctc ctccagaaga cggatgagct 5880
cggcgacagt gtcgcgcacc tcgcgctcaa aggctacagg ggcctcttct tcttcttcaa 5940
tctcctcttc cataagggcc tccccttctt cttcttctgg cggcggtggg ggagggggga 6000
cacggcggcg acgacggcgc accgggaggc ggtcgacaaa gcgctcgatc atctccccgc 6060
ggcgacggcg catggtctcg gtgacggcgc ggccgttctc gcgggggcgc agttggaaga 6120
cgccgcccgt catgtcccgg ttatgggttg gcggggggct gccatgcggc agggatacgg 6180
cgctaacgat gcatctcaac aattgttgtg taggtactcc gccgccgagg gacctgagcg 6240
agtccgcatc gaccggatcg gaaaacctct cgagaaaggc gtctaaccag tcacagtcgc 6300
aaggtaggct gagcaccgtg gcgggcggca gcgggcggcg gtcggggttg tttctggcgg 6360
aggtgctgct gatgatgtaa ttaaagtagg cggtcttgag acggcggatg gtcgacagaa 6420
gcaccatgtc cttgggtccg gcctgctgaa tgcgcaggcg gtcggccatg ccccaggctt 6480
cgttttgaca tcggcgcagg tctttgtagt agtcttgcat gagcctttct accggcactt 6540
cttcttctcc ttcctcttgt cctgcatctc ttgcatctat cgctgcggcg gcggcggagt 6600
ttggccgtag gtggcgccct cttcctccca tgcgtgtgac cccgaagccc ctcatcggct 6660
gaagcagggc taggtcggcg acaacgcgct cggctaatat ggcctgctgc acctgcgtga 6720
gggtagactg gaagtcatcc atgtccacaa agcggtggta tgcgcccgtg ttgatggtgt 6780
aagtgcagtt ggccataacg gaccagttaa cggtctggtg acccggctgc gagagctcgg 6840
tgtacctgag acgcgagtaa gccctcgagt caaatacgta gtcgttgcaa gtccgcacca 6900
ggtactggta tcccaccaaa aagtgcggcg gcggctggcg gtagaggggc cagcgtaggg 6960
tggccggggc tccgggggcg agatcttcca acataaggcg atgatatccg tagatgtacc 7020
tggacatcca ggtgatgccg gcggcggtgg tggaggcgcg cggaaagtcg cggacgcggt 7080
tccagatgtt gcgcagcggc aaaaagtgct ccatggtcgg gacgctctgg ccggtcaggc 7140

gcgcgcaatc gttgacgctc tagcgtgcaa aaggagagcc tgtaagcggg cactcttccg 7200
tggtctggtg gataaattcg caagggtatc atggcggacg accggggttc gagccccgta 7260
tccggccgtc cgccgtgatc catgcggtta ccgcccgcgt gtcgaaccca ggtgtgcgac 7320
gtcagacaac gggggagtgc tccttttggc ttccttccag gcgcggcggc tgctgcgcta 7380
gcttttttgg ccactggccg cgcgcagcgt aagcggttag gctggaaagc gaaagcatta 7440
agtggctcgc tccctgtagc cggagggtta ttttccaagg gttgagtcgc gggacccccg 7500
gttcgagtct cggaccggcc ggactgcggc gaacgggggt ttgcctcccc gtcatgcaag 7560
accccgcttg caaattcctc cggaaacagg gacgagcccc ttttttgctt ttcccagatg 7620
catccggtgc tgcggcagat gcgcccccct cctcagcagc ggcaagagca agagcagcgg 7680
cagacatgca gggcaccctc ccctcctcct accgcgtcag gaggggcgac atccgcggtt 7740
gacgcggcag cagatggtga ttacgaaccc ccgcggcgcc gggcccggca ctacctggac 7800
ttggaggagg gcgagggcct ggcgcggcta ggagcgccct ctcctgagcg gcacccaagg 7860
gtgcagctga agcgtgatac gcgtgaggcg tacgtgccgc ggcagaacct gtttcgcgac 7920
cgcgagggag aggagcccga ggagatgcgg gatcgaaagt tccacgcagg gcgcgagctg 7980
cggcatggcc tgaatcgcga gcggttgctg cgcgaggagg actttgagcc cgacgcgcga 8040
accgggatta gtcccgcgcg cgcacacgtg gcggccgccg acctggtaac cgcatacgag 8100
cagacggtga accaggagat taactttcaa aaaagcttta acaaccacgt gcgtacgctt 8160
gtggcgcgcg aggaggtggc tataggactg atgcatctgt gggactttgt aagcgcgctg 8220
gagcaaaacc caaatagcaa gccgctcatg gcgcagctgt tccttatagt gcagcacagc 8280
agggacaacg aggcattcag ggatgcgctg ctaaacatag tagagcccga gggccgctgg 8340
ctgctcgatt tgataaacat cctgcagagc atagtggtgc aggagcgcag cttgagcctg 8400
gctgacaagg tggccgccat caactattcc atgcttagcc tgggcaagtt ttacgcccgc 8460
aagatatacc atacccctta cgttcccata gacaaggagg taaagatcga ggggttctac 8520
atgcgcatgg cgctgaaggt gcttaccttg agcgacgacc tgggcgttta tcgcaacgag 8580
cgcatccaca aggccgtgag cgtgagccgg cggcgcgagc tcagcgaccg cgagctgatg 8640
cacagcctgc aaagggccct ggctggcacg ggcagcggcg atagagaggc cgagtcctac 8700
tttgacgcgg gcgctgacct gcgctgggcc ccaagccgac gcgccctgga ggcagctggg 8760
gccggacctg ggctggcggt ggcacccgcg cgcgctggca acgtcggcgg cgtggaggaa 8820
tatgacgagg acgatgagta cgagccagag gacggcgagt actaagcggt gatgtttctg 8880
atcagatgat gcaagacgca acggacccgg cggtgcgggc ggcgctgcag agccagccgt 8940
ccggccttaa ctccacggac gactggcgcc aggtcatgga ccgcatcatg tcgctgactg 9000
cgcgcaatcc tgacgcgttc cggcagcagc cgcaggccaa ccggctctcc gcaattctgg 9060
aagcggtggt cccggcgcgc gcaaacccca cgcacgagaa ggtgctggcg atcgtaaacg 9120

cgctggccga aaacagggcc atccggcccg acgaggccgg cctggtctac gacgcgctgc 9180

ttcagcgcgt ggctcgttac aacagcggca acgtgcagac caacctggac cggctggtgg 9240

gggatgtgcg cgaggccgtg gcgcagcgtg agcgcgcgca gcagcagggc aacctgggct 9300

ccatggttgc actaaacgcc ttcctgagta cacagcccgc caacgtgccg cggggacagg 9360

aggactacac caactttgtg agcgcactgc ggctaatggt gactgagaca ccgcaaagtg 9420

aggtgtacca gtctgggcca gactattttt tccagaccag tagacaaggc ctgcagaccg 9480

taaacctgag ccaggctttc aaaaacttgc aggggctgtg gggggtgcgg gctcccacag 9540

gcgaccgcgc gaccgtgtct agcttgctga cgcccaactc gcgcctgttg ctgctgctaa 9600

tagcgccctt cacggacagt ggcagcgtgt cccgggacac atacctaggt cacttgctga 9660

cactgtaccg cgaggccata ggtcaggcgc atgtggacga gcatactttc caggagatta 9720

caagtgtcag ccgcgcgctg gggcaggagg acacgggcag cctggaggca accctaaact 9780

acctgctgac caaccggcgg cagaagatcc cctcgttgca cagtttaaac agcgaggagg 9840

agcgcatttt gcgctacgtg cagcagagcg tgagccttaa cctgatgcgc gacggggtaa 9900

cgcccagcgt ggcgctggac atgaccgcgc gcaacatgga accgggcatg tatgcctcaa 9960

accggccgtt tatcaaccgc ctaatggact acttgcatcg cgcggccgcc gtgaaccccg 10020

agtatttcac caatgccatc ttgaacccgc actggctacc gccccctggt ttctacaccg 10080

ggggattcga ggtgcccgag ggtaacgatg gattcctctg ggacgacata gacgacagcg 10140

tgttttcccc gcaaccgcag accctgctag agttgcaaca gcgcgagcag gcagaggcgg 10200

cgctgcgaaa ggaaagcttc cgcaggccaa gcagcttgtc cgatctaggc gctgcggccc 10260

cgcggtcaga tgctagtagc ccatttccaa gcttgatagg gtctcttacc agcactcgca 10320

ccacccgccc gcgcctgctg ggcgaggagg agtacctaaa caactcgctg ctgcagccgc 10380

agcgcgaaaa aaacctgcct ccggcatttc ccaacaacgg gatagagagc ctagtggaca 10440

agatgagtag atggaagacg tacgcgcagg agcacaggga cgtgccaggc ccgcgcccgc 10500

ccacccgtcg tcaaaggcac gaccgtcagc ggggtctggt gtgggaggac gatgactcgg 10560

cagacgacag cagcgtcctg gatttgggag ggagtggcaa cccgtttgcg caccttcgcc 10620

ccaggctggg gagaatgttt taaaaaaaaa aaaagcatga tgcaaaataa aaaactcacc 10680

aaggccatgg caccgagcgt tggttttctt gtattcccct tagtatgcgg cgcgcggcga 10740

tgtatgagga aggtcctcct ccctcctacg agagtgtggt gagcgcggcg ccagtggcgg 10800

cggcgctggg ttctcccttc gatgctcccc tggacccgcc gtttgtgcct ccgcggtacc 10860

tgcggcctac cggggggaga aacagcatcc gttactctga gttggcaccc ctattcgaca 10920

ccacccgtgt gtacctggtg gacaacaagt caacggatgt ggcatccctg aactaccaga 10980

acgaccacag caactttctg accacggtca ttcaaaacaa tgactacagc ccgggggagg 11040

caagcacaca gaccatcaat cttgacgacc ggtcgcactg gggcggcgac ctgaaaacca 11100

tcctgcatac caacatgcca aatgtgaacg agttcatgtt taccaataag tttaaggcgc 11160

gggtgatggt gtcgcgcttg cctactaagg acaatcaggt ggagctgaaa tacgagtggg 11220

tggagttcac gctgcccgag ggcaactact ccgagaccat gaccatagac cttatgaaca 11280

acgcgatcgt ggagcactac ttgaaagtgg gcagacagaa cggggttctg gaaagcgaca 11340

tcggggtaaa gtttgacacc cgcaacttca gactggggtt tgaccccgtc actggtcttg 11400

tcatgcctgg ggtatataca aacgaagcct tccatccaga catcattttg ctgccaggat 11460

gcggggtgga cttcacccac agccgcctga gcaacttgtt gggcatccgc aagcggcaac 11520

ccttccagga gggctttagg atcacctacg atgatctgga gggtggtaac attcccgcac 11580

tgttggatgt ggacgcctac caggcgagct tgaaagatga caccgaacag ggcgggggtg 11640

gcgcaggcgg cagcaacagc agtggcagcg gcgcggaaga gaactccaac gcggcagccg 11700

cggcaatgca gccggtggag gacatgaacg atcatgccat tcgcggcgac acctttgcca 11760

cacgggctga ggagaagcgc gctgaggccg aagcagcggc cgaagctgcc gcccccgctg 11820

cgcaacccga ggtcgagaag cctcagaaga aaccggtgat caaacccctg acagaggaca 11880

gcaagaaacg cagttacaac ctaataagca atgacagcac cttcacccag taccgcagct 11940

ggtaccttgc atacaactac ggcgaccctc agaccggaat ccgctcatgg accctgcttt 12000

gcactcctga cgtaacctgc ggctcggagc aggtctactg gtcgttgcca gacatgatgc 12060

aagaccccgt gaccttccgc tccacgcgcc agatcagcaa ctttccggtg gtgggcgccg 12120

agctgttgcc cgtgcactcc aagagcttct acaacgacca ggccgtctac tcccaactca 12180

tccgccagtt tacctctctg acccacgtgt tcaatcgctt tcccgagaac cagattttgg 12240

cgcgcccgcc agcccccacc atcaccaccg tcagtgaaaa cgttcctgct ctcacagatc 12300

acgggacgct accgctgcgc aacagcatcg gaggagtcca gcgagtgacc attactgacg 12360

ccagacgccg cacctgcccc tacgtttaca aggccctggg catagtctcg ccgcgcgtcc 12420

tatcgagccg cactttttga gcaagcatgt ccatccttat atcgcccagc aataacacag 12480

gctggggcct gcgcttccca agcaagatgt ttggcggggc caagaagcgc tccgaccaac 12540

acccagtgcg cgtgcgcggg cactaccgcg cgccctgggg cgcgcacaaa cgcggccgca 12600

ctgggcgcac caccgtcgat gacgccatcg acgcggtggt ggaggaggcg cgcaactaca 12660

cgcccacgcc gccaccagtg tccacagtgg acgcggccat tcagaccgtg gtgcgcggag 12720

cccggcgcta tgctaaaatg aagagacggc ggaggcgcgt agcacgtcgc caccgccgcc 12780

gacccggcac tgccgcccaa cgcgcggcgg cggccctgct taaccgcgca cgtcgcaccg 12840

gccgacgggc ggccatgcgg gccgctcgaa ggctggccgc gggtattgtc actgtgcccc 12900

ccaggtccag gcgacgagcg gccgccgcag cagccgcggc cattagtgct atgactcagg 12960

gtcgcagggg caacgtgtat tgggtgcgcg actcggttag cggcctgcgc gtgcccgtgc 13020

gcacccgccc cccgcgcaac tagattgcaa gaaaaaacta cttagactcg tactgttgta 13080

tgtatccagc ggcggcggcg cgcaacgaag ctatgtccaa gcgcaaaatc aaagaagaga 13140

tgctccaggt catcgcgccg gagatctatg gccccccgaa gaaggaagag caggattaca 13200

agccccgaaa gctaaagcgg gtcaaaaaga aaaagaaaga tgatgatgat gaacttgacg 13260

acgaggtgga actgctgcac gctaccgcgc ccaggcgacg ggtacagtgg aaaggtcgac 13320

gcgtaaaacg tgttttgcga cccggcacca ccgtagtctt tacgcccggt gagcgctcca 13380

cccgcaccta caagcgcgtg tatgatgagg tgtacggcga cgaggacctg cttgagcagg 13440

ccaacgagcg cctcggggag tttgcctacg gaaagcggca taaggacatg ctggcgttgc 13500

cgctggacga gggcaaccca acacctagcc taaagcccgt aacactgcag caggtgctgc 13560

ccgcgcttgc accgtccgaa gaaaagcgcg gcctaaagcg cgagtctggt gacttggcac 13620

ccaccgtgca gctgatggta cccaagcgcc agcgactgga agatgtcttg gaaaaaatga 13680

ccgtggaacc tgggctggag cccgaggtcc gcgtgcggcc aatcaagcag gtggcgccgg 13740

gactgggcgt gcagaccgtg gacgttcaga tacccactac cagtagcacc agtattgcca 13800

ccgccacaga gggcatggag acacaaacgt ccccggttgc ctcagcggtg gcggatgccg 13860

cggtgcaggc ggtcgctgcg gccgcgtcca agacctctac ggaggtgcaa acggacccgt 13920

ggatgtttcg cgtttcagcc ccccggcgcc cgcgccgttc gaggaagtac ggcgccgcca 13980

gcgcgctact gcccgaatat gccctacatc cttccattgc gcctaccccc ggctatcgtg 14040

gctacaccta ccgccccaga agacgagcaa ctacccgacg ccgaaccacc actggaaccc 14100

gccgccgccg tcgccgtcgc cagcccgtgc tggccccgat ttccgtgcgc agggtggctc 14160

gcgaaggagg caggaccctg gtgctgccaa cagcgcgcta ccaccccagc atcgtttaaa 14220

agccggtctt tgtggttctt gcagatatgg ccctcacctg ccgcctccgt ttcccggtgc 14280

cgggattccg aggaagaatg caccgtagga ggggcatggc cggccacggc ctgacgggcg 14340

gcatgcgtcg tgcgcaccac cggcggcggc gcgcgtcgca ccgtcgcatg cgcggcggta 14400

tcctgcccct ccttattcca ctgatcgccg cggcgattgg cgccgtgccc ggaattgcat 14460

ccgtggcctt gcaggcgcag agacactgat taaaaacaag ttgcatgtgg aaaaatcaaa 14520

ataaaaagtc tggactctca cgctcgcttg gtcctgtaac tattttgtag aatggaagac 14580

atcaactttg cgtctctggc cccgcgacac ggctcgcgcc cgttcatggg aaactggcaa 14640

gatatcggca ccagcaatat gagcggtggc gccttcagct ggggctcgct gtggagcggc 14700

attaaaaatt tcggttccac cgttaagaac tatggcagca aggcctggaa cagcagcaca 14760

ggccagatgc tgagggataa gttgaaagag caaaatttcc aacaaaaggt ggtagatggc 14820

ctggcctctg gcattagcgg ggtggtggac ctggccaacc aggcagtgca aaataagatt 14880

aacagtaagc ttgatccccg ccctcccgta gaggagcctc caccggccgt ggagacagtg 14940

tctccagagg ggcgtggcga aaagcgtccg cgccccgaca gggaagaaac tctggtgacg 15000

caaatagacg agcctccctc gtacgaggag gcactaaagc aaggcctgcc caccacccgt 15060

cccatcgcgc ccatggctac cggagtgctg ggccagcaca cacccgtaac gctggacctg 15120
cctccccccg ccgacaccca gcagaaacct gtgctgccag gcccgaccgc cgttgttgta 15180
acccgtccta gccgcgcgtc cctgcgccgc gccgccagcg gtccgcgatc gttgcggccc 15240
gtagccagtg gcaactggca aagcacactg aacagcatcg tgggtctggg ggtgcaatcc 15300
ctgaagcgcc gacgatgctt ctgatagcta acgtgtcgta tgtgtgtcat gtatgcgtcc 15360
atgtcgccgc cagaggagct gctgagccgc cgcgcgcccg ctttccaaga tggctacccc 15420
ttcgatgatg ccgcagtggt cttacatgca catctcgggc caggacgcct cggagtacct 15480
gagccccggg ctggtgcagt ttgcccgcgc caccgagacg tacttcagcc tgaataacaa 15540
gtttagaaac cccacggtgg cgcctacgca cgacgtgacc acagaccggt cccagcgttt 15600
gacgctgcgg ttcatccctg tggaccgtga ggatactgcg tactcgtaca aggcgcggtt 15660
caccctagct gtgggtgata accgtgtgct ggacatggct tccacgtact ttgacatccg 15720
cggcgtgctg gacaggggcc ctacttttaa gccctactct ggcactgcct acaacgccct 15780
ggctcccaag ggtgccccaa atccttgcga atgggatgaa gctgctactg ctcttgaaat 15840
aaacctagaa gaagaggacg atgacaacga agacgaagta gacgagcaag ctgagcagca 15900
aaaaactcac gtatttgggc aggcgcctta ttctggtata aatattacaa aggagggtat 15960
tcaaataggt gtcgaaggtc aaacacctaa atatgccgat aaaacatttc aacctgaacc 16020
tcaaatagga gaatctcagt ggtacgaaac agaaattaat catgcagctg ggagagtcct 16080
aaaaaagact accccaatga aaccatgtta cggttcatat gcaaaaccca caaatgaaaa 16140
tggagggcaa ggcattcttg taaagcaaca aaatggaaag ctagaaagtc aagtggaaat 16200
gcaatttttc tcaactactg aggcagccgc aggcaatggt gataacttga ctcctaaagt 16260
ggtattgtac agtgaagatg tagatataga aaccccagac actcatattt cttacatgcc 16320
cactattaag gaaggtaact cacgagaact aatgggccaa caatctatgc ccaacaggcc 16380
taattacatt gcttttaggg acaattttat tggtctaatg tattacaaca gcacgggtaa 16440
tatgggtgtt ctggcgggcc aagcatcgca gttgaatgct gttgtagatt tgcaagacag 16500
aaacacagag ctttcatacc agcttttgct tgattccatt ggtgatagaa ccaggtactt 16560
ttctatgtgg aatcaggctg ttgacagcta tgatccagat gttagaatta ttgaaaatca 16620
tggaactgaa gatgaacttc caaattactg ctttccactg ggaggtgtga ttaatacaga 16680
gactcttacc aaggtaaaac ctaaaacagg tcaggaaaat ggatgggaaa aagatgctac 16740
agaattttca gataaaaatg aaataagagt tggaaataat tttgccatgg aaatcaatct 16800
aaatgccaac ctgtggagaa atttcctgta ctccaacata gcgctgtatt tgcccgacaa 16860
gctaaagtac agtccttcca acgtaaaaat ttctgataac ccaaacacct acgactacat 16920
gaacaagcga gtggtggctc ccgggctagt ggactgctac attaaccttg gagcacgctg 16980
gtcccttgac tatatggaca acgtcaaccc atttaaccac caccgcaatg ctggcctgcg 17040

ctaccgctca atgttgctgg gcaatggtcg ctatgtgccc ttccacatcc aggtgcctca 17100

gaagttcttt gccattaaaa acctccttct cctgccgggc tcatacacct acgagtggaa 17160

cttcaggaag gatgttaaca tggttctgca gagctcccta ggaaatgacc taagggttga 17220

cggagccagc attaagtttg atagcatttg cctttacgcc accttcttcc ccatggccca 17280

caacaccgcc tccacgcttg aggccatgct tagaaacgac accaacgacc agtcctttaa 17340

cgactatctc tccgccgcca acatgctcta ccctataccc gccaacgcta ccaacgtgcc 17400

catatccatc ccctcccgca actgggcggc tttccgcggc tgggccttca cgcgccttaa 17460

gactaaggaa accccatcac tgggctcggg ctacgaccct tattacacct actctggctc 17520

tataccctac ctagatggaa ccttttacct caaccacacc tttaagaagg tggccattac 17580

ctttgactct tctgtcagct ggcctggcaa tgaccgcctg cttaccccca acgagtttga 17640

aattaagcgc tcagttgacg gggagggtta caacgttgcc cagtgtaaca tgaccaaaga 17700

ctggttcctg gtacaaatgc tagctaacta taacattggc taccagggct tctatatccc 17760

agagagctac aaggaccgca tgtactcctt ctttagaaac ttccagccca tgagccgtca 17820

ggtggtggat gatactaaat acaaggacta ccaacaggtg ggcatcctac accaacacaa 17880

caactctgga tttgttggct accttgcccc caccatgcgc gaaggacagg cctaccctgc 17940

taacttcccc tatccgctta taggcaagac cgcagttgac agcattaccc agaaaaagtt 18000

tctttgcgat cgcacccttt ggcgcatccc attctccagt aactttatgt ccatgggcgc 18060

actcacagac ctgggccaaa accttctcta cgccaactcc gcccacgcgc tagacatgac 18120

ttttgaggtg gatcccatgg acgagcccac ccttctttat gttttgtttg aagtctttga 18180

cgtggtccgt gtgcaccagc cgcaccgcgg cgtcatcgaa accgtgtacc tgcgcacgcc 18240

cttctcggcc ggcaacgcca caacataaag aagcaagcaa catcaacaac agctgccgcc 18300

atgggctcca gtgagcagga actgaaagcc attgtcaaag atcttggttg tgggccatat 18360

tttttgggca cctatgacaa gcgctttcca ggctttgttt ctccacacaa gctcgcctgc 18420

gccatagtca atacggccgg tcgcgagact gggggcgtac actggatggc ctttgcctgg 18480

aacccgcact caaaaacatg ctacctcttt gagccctttg gcttttctga ccagcgactc 18540

aagcaggttt accagtttga gtacgagtca ctcctgcgcc gtagcgccat tgcttcttcc 18600

cccgaccgct gtataacgct ggaaaagtcc acccaaagcg tacaggggcc caactcggcc 18660

gcctgtggac tattctgctg catgtttctc cacgcctttg ccaactggcc ccaaactccc 18720

atggatcaca accccaccat gaaccttatt accggggtac ccaactccat gctcaacagt 18780

ccccaggtac agcccaccct gcgtcgcaac caggaacagc tctacagctt cctggagcgc 18840

cactcgccct acttccgcag ccacagtgcg cagattagga gcgccacttc tttttgtcac 18900

ttgaaaaaca tgtaaaaata atgtactaga gacactttca ataaaggcaa atgcttttat 18960

ttgtacactc tcgggtgatt atttaccccc acccttgccg tctgcgccgt ttaaaaatca 19020

aaggggttct gccgcgcatc gctatgcgcc actggcaggg acacgttgcg atactggtgt 19080
ttagtgctcc acttaaactc aggcacaacc atccgcggca gctcggtgaa gttttcactc 19140
cacaggctgc gcaccatcac caacgcgttt agcaggtcgg gcgccgatat cttgaagtcg 19200
cagttggggc ctccgccctg cgcgcgcgag ttgcgataca cagggttgca gcactggaac 19260
actatcagcg ccgggtggtg cacgctggcc agcacgctct tgtcggagat cagatccgcg 19320
tccaggtcct ccgcgttgct cagggcgaac ggagtcaact ttggtagctg ccttcccaaa 19380
aagggcgcgt gcccaggctt tgagttgcac tcgcaccgta gtggcatcaa aaggtgaccg 19440
tgcccggtct gggcgttagg atacagcgcc tgcataaaag ccttgatctg cttaaaagcc 19500
acctgagcct ttgcgccttc agagaagaac atgccgcaag acttgccgga aaactgattg 19560
gccggacagg ccgcgtcgtg cacgcagcac cttgcgtcgg tgttggagat ctgcaccaca 19620
tttcggcccc accggttctt cacgatcttg gccttgctag actgctcctt cagcgcgcgc 19680
tgcccgtttt cgctcgtcac atccatttca atcacgtgct ccttatttat cataatgctt 19740
ccgtgtagac acttaagctc gccttcgatc tcagcgcagc ggtgcagcca caacgcgcag 19800
cccgtgggct cgtgatgctt gtaggtcacc tctgcaaacg actgcaggta cgcctgcagg 19860
aatcgcccca tcatcgtcac aaaggtcttg ttgctggtga aggtcagctg caacccgcgg 19920
tgctcctcgt tcagccaggt cttgcatacg gccgccagag cttccacttg gtcaggcagt 19980
agtttgaagt tcgcctttag atcgttatcc acgtggtact tgtccatcag cgcgcgcgca 20040
gcctccatgc ccttctccca cgcagacacg atcggcacac tcagcgggtt catcaccgta 20100
atttcacttt ccgcttcgct gggctcttcc tcttcctctt gcgtccgcat accacgcgcc 20160
actgggtcgt cttcattcag ccgccgcact gtgcgcttac ctcctttgcc atgcttgatt 20220
agcaccggtg ggttgctgaa acccaccatt tgtagcgcca catcttctct ttcttcctcg 20280
ctgtccacga ttacctctgg tgatggcggg cgctcgggct tgggagaagg gcgcttcttt 20340
ttcttcttgg gcgcaatggc caaatccgcc gccgaggtcg atggccgcgg gctgggtgtg 20400
cgcggcacca gcgcgtcttg tgatgagtct tcctcgtcct cggactcgat acgccgcctc 20460
atccgctttt ttgggggcgc ccggggaggc ggcggcgacg gggacgggga cgacacgtcc 20520
tccatggttg ggggacgtcg cgccgcaccg cgtccgcgct cgggggtggt ttcgcgctgc 20580
tcctcttccc gactggccat ttccttctcc tataggcaga aaaagatcat ggagtcagtc 20640
gagaagaagg acagcctaac cgccccctct gagttcgcca ccaccgcctc caccgatgcc 20700
gccaacgcgc ctaccacctt ccccgtcgag gcacccccgc ttgaggagga ggaagtgatt 20760
atcgagcagg acccaggttt tgtaagcgaa gacgacgagg accgctcagt accaacagag 20820
gataaaaagc aagaccagga caacgcagag gcaaacgagg aacaagtcgg gcggggggac 20880
gaaaggcatg gcgactacct agatgtggga gacgacgtgc tgttgaagca tctgcagcgc 20940
cagtgcgcca ttatctgcga cgcgttgcaa gagcgcagcg atgtgcccct cgccatagcg 21000

gatgtcagcc ttgcctacga acgccaccta ttctcaccgc gcgtaccccc caaacgccaa 21060

gaaaacggca catgcgagcc caacccgcgc ctcaacttct accccgtatt tgccgtgcca 21120

gaggtgcttg ccacctatca catctttttc caaaactgca agatacccct atcctgccgt 21180

gccaaccgca gccgagcgga caagcagctg gccttgcggc agggcgctgt catacctgat 21240

atcgcctcgc tcaacgaagt gccaaaaatc tttgagggtc ttggacgcga cgagaagcgc 21300

gcggcaaacg ctctgcaaca ggaaaacagc gaaaatgaaa gtcactctgg agtgttggtg 21360

gaactcgagg gtgacaacgc gcgcctagcc gtactaaaac gcagcatcga ggtcacccac 21420

tttgcctacc cggcacttaa cctacccccc aaggtcatga gcacagtcat gagtgagctg 21480

atcgtgcgcc gtgcgcagcc cctggagagg gatgcaaatt tgcaagaaca aacagaggag 21540

ggcctacccg cagttggcga cgagcagcta gcgcgctggc ttcaaacgcg cgagcctgcc 21600

gacttggagg agcgacgcaa actaatgatg gccgcagtgc tcgttaccgt ggagcttgag 21660

tgcatgcagc ggttctttgc tgacccggag atgcagcgca agctagagga aacattgcac 21720

tacacctttc gacagggcta cgtacgccag gcctgcaaga tctccaacgt ggagctctgc 21780

aacctggtct cctaccttgg aattttgcac gaaaaccgcc ttgggcaaaa cgtgcttcat 21840

tccacgctca agggcgaggc gcgccgcgac tacgtccgcg actgcgttta cttatttcta 21900

tgctacacct ggcagacggc catgggcgtt tggcagcagt gcttggagga gtgcaacctc 21960

aaggagctgc agaaactgct aaagcaaaac ttgaaggacc tatggacggc cttcaacgag 22020

cgctccgtgg ccgcgcacct ggcggacatc attttccccg aacgcctgct taaaaccctg 22080

caacagggtc tgccagactt caccagtcaa agcatgttgc agaactttag gaactttatc 22140

ctagagcgct caggaatctt gcccgccacc tgctgtgcac ttcctagcga ctttgtgccc 22200

attaagtacc gcgaatgccc tccgccgctt tggggccact gctaccttct gcagctagcc 22260

aactaccttg cctaccactc tgacataatg gaagacgtga gcggtgacgg tctactggag 22320

tgtcactgtc gctgcaacct atgcaccccg caccgctccc tggtttgcaa ttcgcagctg 22380

cttaacgaaa gtcaaattat cggtaccttt gagctgcagg gtccctcgcc tgacgaaaag 22440

tccgcggctc cggggttgaa actcactccg gggctgtgga cgtcggctta ccttcgcaaa 22500

tttgtacctg aggactacca cgcccacgag attaggttct acgaagacca atcccgcccg 22560

cctaatgcgg agcttaccgc ctgcgtcatt acccagggcc acattcttgg ccaattgcaa 22620

gccatcaaca aagcccgcca agagtttctg ctacgaaagg gacggggggt ttacttggac 22680

ccccagtccg gcgaggagct caacccaatc cccccgccgc cgcagcccta tcagcagcag 22740

ccgcgggccc ttgcttccca ggatggcacc caaaaagaag ctgcagctgc cgccgccacc 22800

cacggacgag gaggaatact gggacagtca ggcagaggag gttttggacg aggaggagga 22860

ggacatgatg gaagactggg agagcctaga cgaggaagct tccgaggtcg aagaggtgtc 22920

agacgaaaca ccgtcaccct cggtcgcatt cccctcgccg gcgccccaga aatcggcaac 22980

cggttccagc atggctacaa cctccgctcc tcaggcgccg ccggcactgc ccgttcgccg 23040

acccaaccgt agatgggaca ccactggaac cagggccggt aagtccaagc agccgccgcc 23100

gttagcccaa gagcaacaac agcgccaagg ctaccgctca tggcgcgggc acaagaacgc 23160

catagttgct tgcttgcaag actgtggggg caacatctcc ttcgcccgcc gctttcttct 23220

ctaccatcac ggcgtggcct tcccccgtaa catcctgcat tactaccgtc atctctacag 23280

cccatactgc accggcggca gcggcagcaa cagcagcggc cacacagaag caaaggcgac 23340

cggatagcaa gactctgaca aagcccaaga aatccacagc ggcggcagca gcaggaggag 23400

gagcgctgcg tctggcgccc aacgaacccg tatcgacccg cgagcttaga aacaggattt 23460

ttcccactct gtatgctata tttcaacaga gcaggggcca agaacaagag ctgaaaataa 23520

aaaacaggtc tctgcgatcc ctcacccgca gctgcctgta tcacaaaagc gaagatcagc 23580

ttcggcgcac gctggaagac gcggaggctc tcttcagtaa atactgcgcg ctgactctta 23640

aggactagtt tcgcgccctt tctcaaattt aagcgcgaaa actacgtcat ctccagcggc 23700

cacacccggc gccagcacct gttgtcagcg ccattatgag caaggaaatt cccacgccct 23760

acatgtggag ttaccagcca caaatgggac ttgcggctgg agctgcccaa gactactcaa 23820

cccgaataaa ctacatgagc gcgggacccc acatgatatc ccgggtcaac ggaatacgcg 23880

cccaccgaaa ccgaattctc ttggaacagg cggctattac caccacacct cgtaataacc 23940

ttaatccccg tagttggccc gctgccctgg tgtaccagga aagtcccgct cccaccactg 24000

tggtacttcc cagagacgcc caggccgaag ttcagatgac taactcaggg gcgcagcttg 24060

cgggcggctt tcgtcacagg gtgcggtcgc ccgggcaggg tataactcac ctgacaatca 24120

gagggcgagg tattcagctc aacgacgagt cggtgagctc ctcgcttggt ctccgtccgg 24180

acgggacatt tcagatcggc ggcgccggcc gtccttcatt cacgcctcgt caggcaatcc 24240

taactctgca gacctcgtcc tctgagccgc gctctggagg cattggaact ctgcaattta 24300

ttgaggagtt tgtgccatcg gtctacttta accccttctc gggacctccc ggccactatc 24360

cggatcaatt tattcctaac tttgacgcgg taaaggactc ggcggacggc tacgactgaa 24420

tgttaagtgg agaggcagag caactgcgcc tgaaacacct ggtccactgt cgccgccaca 24480

agtgctttgc ccgcgactcc ggtgagtttt gctactttga attgcccgag gatcatatcg 24540

agggcccggc gcacggcgtc cggcttaccg cccagggaga gcttgcccgt agcctgattc 24600

gggagtttac ccagcgcccc ctgctagttg agcgggacag gggaccctgt gttctcactg 24660

tgatttgcaa ctgtcctaac cttggattac atcaagatcc tctagttaat gtcgacttcg 24720

cgatggatcc attaactaat aaaaaaaaat aataaagcat cacttactta aaatcagtta 24780

gcaaatttct gtccagttta ttcagcagca cctccttgcc ctcctcccag ctctggtatt 24840

gcagcttcct cctggctgca aactttctcc acaatctaaa tggaatgtca gtttcctcct 24900

gttcctgtcc atccgcaccc actatcttca tgttgttgca gatgaagcgc gcaagaccgt 24960

ctgaagatac cttcaacccc gtgtatccat atgacacgga aaccggtcct ccaactgtgc 25020

cttttcttac tcctcccttt gtatccccca atgggtttca agagagtccc cctggagttc 25080

ttactttaaa atgtttaacc ccactaacaa ccacaggcgg atctctacag ctaaaagtgg 25140

gagggggact tacagtggat gacactgatg gtaccttaca agaaaacata cgtgctacag 25200

cacccattac taaaaataat cactctgtag aactatccat tggaaatgga ttagaaactc 25260

aaaacaataa actatgtgcc aaattgggaa atgggttaaa atttaacaac ggtgacattt 25320

gtataaagga tagtattaac accttatgga ctggaataaa ccctccacct aactgtcaaa 25380

ttgtggaaaa cactaataca aatgatggca aacttacttt agtattagta aaaaacggag 25440

ggcttgttaa tggctacgtg tctctagttg gtgtatcaga cactgtgaac caaatgttca 25500

cacaaaagac agcaaacatc caattaagat tatattttga ctcttctgga aatctattaa 25560

ctgatgaatc agacttaaaa attccactta aaaataaatc ttctacagcg accagtgaaa 25620

ctgtagccag cagcaaagcc tttatgccaa gtactacagc ttatcccttc aacaccacta 25680

ctagggatag tgaaaactac attcatggaa tatgttacta catgactagt tatgatagaa 25740

gtctatttcc cttgaacatt tctataatgc taaacagccg tatgatttct tccaatgttg 25800

cctatgccat acaatttgaa tggaatctaa atgcaagtga atctccagaa agcaacatag 25860

ctacgctgac cacatccccc tttttctttt cttacattac agaagacgac aactaaaatg 25920

cccaagaata aagaatcgtt tgtgttatgt ttcaacgtgt ttatttttca attgcagaaa 25980

atttcaagtc atttttcatt cagtagtata gccccaccac cacatagctt atacagatca 26040

ccgtacctta atcaaactca cagaacccta gtattcaacc tgccacctcc ctcccaacac 26100

acagagtaca cagtcctttc tccccggctg gccttaaaaa gcatcatatc atgggtaaca 26160

gacatattct taggtgttat attccacacg gtttcctgtc gagccaaacg ctcatcagtg 26220

atattaataa actccccggg cagctcactt aagttcatgt cgctgtccag ctgctgagcc 26280

acaggctgct gtccaacttg cggttgctta acgggcggcg aaggagaagt ccacgcctac 26340

atgggggtag agtcataatc gtgcatcagg atagggcggt ggtgctgcag cagcgcgcga 26400

ataaactgct gccgccgccg ctccgtcctg caggaataca acatggcagt ggtctcctca 26460

gcgatgattc gcaccgcccg cagcataagg cgccttgtcc tccgggcaca gcagcgcacc 26520

ctgatctcac ttaaatcagc acagtaactg cagcacagca ccacaatatt gttcaaaatc 26580

ccacagtgca aggcgctgta tccaaagctc atggcgggga ccacagaacc cacgtggcca 26640

tcataccaca agcgcaggta gattaagtgg cgacccctca taaacacgct ggacataaac 26700

attacctctt ttggcatgtt gtaattcacc acctcccggt accatataaa cctctgatta 26760

aacatggcgc catccaccac catcctaaac cagctggcca aaacctgccc gccggctata 26820

cactgcaggg aaccgggact ggaacaatga cagtggagag cccaggactc gtaaccatgg 26880

atcatcatgc tcgtcatgat atcaatgttg gcacaacaca ggcacacgtg catacacttc 26940

ctcaggatta caagctcctc ccgcgttaga accatatccc agggaacaac ccattcctga 27000
atcagcgtaa atcccacact gcagggaaga cctcgcacgt aactcacgtt gtgcattgtc 27060
aaagtgttac attcgggcag cagcggatga tcctccagta tggtagcgcg ggtttctgtc 27120
tcaaaaggag gtagacgatc cctactgtac ggagtgcgcc gagacaaccg agatcgtgtt 27180
ggtcgtagtg tcatgccaaa tggaacgccg gacgtagtca tatttcctga agcaaaacca 27240
ggtgcgggcg tgacaaacag atctgcgtct ccggtctcgc cgcttagatc gctctgtgta 27300
gtagttgtag tatatccact ctctcaaagc atccaggcgc cccctggctt cgggttctat 27360
gtaaactcct tcatgcgccg ctgccctgat aacatccacc accgcagaat aagccacacc 27420
cagccaacct acacattcgt tctgcgagtc acacacggga ggagcgggaa gagctggaag 27480
aaccatgttt ttttttttat tccaaaagat tatccaaaac ctcaaaatga agatctatta 27540
agtgaacgcg ctcccctccg gtggcgtggt caaactctac agccaaagaa cagataatgg 27600
catttgtaag atgttgcaca atggcttcca aaaggcaaac ggccctcacg tccaagtgga 27660
cgtaaaggct aaacccttca gggtgaatct cctctataaa cattccagca ccttcaacca 27720
tgcccaaata attctcatct cgccaccttc tcaatatatc tctaagcaaa tcccgaatat 27780
taagtccggc cattgtaaaa atctgctcca gagcgccctc caccttcagc ctcaagcagc 27840
gaatcatgat tgcaaaaatt caggttcctc acagacctgt ataagattca aaagcggaac 27900
attaacaaaa ataccgcgat cccgtaggtc ccttcgcagg gccagctgaa cataatcgtg 27960
caggtctgca cggaccagcg cggccacttc cccgccagga accttgacaa aagaacccac 28020
actgattatg acacgcatac tcggagctat gctaaccagc gtagccccga tgtaagcttt 28080
gttgcatggg cggcgatata aaatgcaagg tgctgctcaa aaaatcaggc aaagcctcgc 28140
gcaaaaaaga aagcacatcg tagtcatgct catgcagata aaggcaggta agctccggaa 28200
ccaccacaga aaaagacacc atttttctct caaacatgtc tgcgggtttc tgcataaaca 28260
caaaataaaa taacaaaaaa acatttaaac attagaagcc tgtcttacaa caggaaaaac 28320
aacccttata agcataagac ggactacggc catgccggcg tgaccgtaaa aaaactggtc 28380
accgtgatta aaaagcacca ccgacagctc ctcggtcatg tccggagtca taatgtaaga 28440
ctcggtaaac acatcaggtt gattcatcgg tcagtgctaa aaagcgaccg aaatagcccg 28500
ggggaataca tacccgcagg cgtagagaca acattacagc ccccatagga ggtataacaa 28560
aattaatagg agagaaaaac acataaacac ctgaaaaacc ctcctgccta ggcaaaatag 28620
caccctcccg ctccagaaca acatacagcg cttcacagcg gcagcctaac agtcagcctt 28680
accagtaaaa aagaaaacct attaaaaaaa caccactcga cacggcacca gctcaatcag 28740
tcacagtgta aaaaagggcc aagtgcagag cgagtatata taggactaaa aaatgacgta 28800
acggttaaag tccacaaaaa acacccagaa aaccgcacgc gaacctacgc ccagaaacga 28860
aagccaaaaa acccacaact tcctcaaatc gtcacttccg ttttcccacg ttacgtaact 28920

tcccatttta agaaaactac aattcccaac acatacaagt tactccgccc taaaacctac 28980

gtcacccgcc ccgttcccac gccccgcgcc acgtcacaaa ctccaccccc tcattatcat 29040

attggcttca atccaaaata aggtatatta ttgatgatgt taattaattt cgaacccggg 29100

gtaccgaatt cctcgagtct agaggagcat gcgacgtcgc aattcgccct atagtgagtc 29160

gtattacaat tcactggccg tcgttttaca acgtcgtgac tgggaaaacc ctggcgttac 29220

ccaacttaat cgccttgcag cacatccccc tttcgccagc tggcgtaata gcgaagaggc 29280

ccgcaccgat cgcccttccc aacagttgcg cagcctgaat ggcgaatgga aattgtaagc 29340

gttaatattt tgttaaaatt cgcgttaaat ttttgttaaa tcagctcatt ttttaaccaa 29400

taggccgaaa tcggcaaaat cccttataaa tcaaaagaat agaccgagat agggttgagt 29460

gttgttccag tttggaacaa gagtccacta ttaaagaacg tggactccaa cgtcaaaggg 29520

cgaaaaaccg tctatcaggg cgatggccca ctacgtgaac catcacccta atcaagtttt 29580

ttggggtcga ggtgccgtaa agcactaaat cggaacccta aagggagccc ccgatttaga 29640

gcttgacggg gaaagccggc gaacgtggcg agaaaggaag ggaagaaagc gaaaggagcg 29700

ggcgctaggg cgctggcaag tgtagcggtc acgctgcgcg taaccaccac acccgccgcg 29760

cttaatgcgc cgctacaggg cgcgtcctga tgcggtattt tctccttacg catctgtgcg 29820

gtatttcaca ccgcatacag gtggcacttt tcggggaaat gtgcgcggaa cccctatttg 29880

tttatttttc taaatacatt caaatatgta tccgctcatg agacaataac cctgataaat 29940

gcttcaataa tattgaaaaa ggaagagtat gagtattcaa catttccgtg tcgcccttat 30000

tccctttttt gcggcatttt gccttcctgt ttttgctcac ccagaaacgc tggtgaaagt 30060

aaaagatgct gaagatcagt tgggtgcacg agtgggttac atcgaactgg atctcaacag 30120

cggtaagatc cttgagagtt ttcgccccga agaacgtttt ccaatgatga gcacttttaa 30180

agttctgcta tgtggcgcgg tattatcccg tattgacgcc gggcaagagc aactcggtcg 30240

ccgcatacac tattctcaga atgacttggt tgagtactca ccagtcacag aaaagcatct 30300

tacggatggc atgacagtaa gagaattatg cagtgctgcc ataaccatga gtgataacac 30360

tgcggccaac ttacttctga caacgatcgg aggaccgaag gagctaaccg cttttttgca 30420

caacatgggg gatcatgtaa ctcgccttga tcgttgggaa ccggagctga atgaagccat 30480

accaaacgac gagcgtgaca ccacgatgcc tgtagcaatg gcaacaacgt tgcgcaaact 30540

attaactggc gaactactta ctctagcttc ccggcaacaa ttaatagact ggatggaggc 30600

ggataaagtt gcaggaccac ttctgcgctc ggcccttccg gctggctggt ttattgctga 30660

taaatctgga gccggtgagc gtgggtctcg cggtatcatt gcagcactgg ggccagatgg 30720

taagccctcc cgtatcgtag ttatctacac gacggggagt caggcaacta tggatgaacg 30780

aaatagacag atcgctgaga taggtgcctc actgattaag cattggtaac tgtcagacca 30840

agtttactca tatatacttt agattgattt aaaacttcat ttttaattta aaaggatcta 30900

ggtgaagatc ctttttgata atctcatgac caaaatccct taacgtgagt tttcgttcca 30960

ctgagcgtca gaccccgtag aaaagatcaa aggatcttct tgagatcctt tttttctgcg 31020

cgtaatctgc tgcttgcaaa caaaaaaacc accgctacca gcggtggttt gtttgccgga 31080

tcaagagcta ccaactcttt ttccgaaggt aactggcttc agcagagcgc agataccaaa 31140

tactgttctt ctagtgtagc cgtagttagg ccaccacttc aagaactctg tagcaccgcc 31200

tacatacctc gctctgctaa tcctgttacc agtggctgct gccagtggcg ataagtcgtg 31260

tcttaccggg ttggactcaa gacgatagtt accggataag gcgcagcggt cgggctgaac 31320

ggggggttcg tgcacacagc ccagcttgga gcgaacgacc tacaccgaac tgagatacct 31380

acagcgtgag ctatgagaaa gcgccacgct tcccgaaggg agaaaggcgg acaggtatcc 31440

ggtaagcggc agggtcggaa caggagagcg cacgagggag cttccagggg gaaacgcctg 31500

gtatctttat agtcctgtcg ggtttcgcca cctctgactt gagcgtcgat ttttgtgatg 31560

ctcgtcaggg gggcggagcc tatggaaaaa cgccagcaac gcggcctttt tacggttcct 31620

ggccttttgc tggccttttg ctcacatgtt ctttcctgcg ttatcccctg attctgtgga 31680

taaccgtatt accgcctttg agtgagctga taccgctcgc cgcagccgaa cgaccgagcg 31740

cagcgagtca gtgagcgagg aagcggaaga gcgcccaata cgcaaaccgc ctctccccgc 31800

gcgttggccg attcattaat gcagctggca cgacaggttt cccgactgga aagcgggcag 31860

tgagcgcaac gcaattaatg tgagttagct cactcattag gcaccccagg ctttacactt 31920

tatgcttccg gctcgtatgt tgtgtggaat tgtgagcgga taacaatttc acacaggaaa 31980

cagctatgac catgattacg ccaagctatt taggtgacac tatagaatac tcaagctagt 32040

taattaacgt taattaacat catcaataat ataccttatt ttggattgaa gccaatatga 32100

taatgagggg gtggagtttg tgacgtggcg cggggcgtgg gaacggggcg ggtgacgtag 32160

tagtgtggcg gaagtgtgat gttgcaagtg tggcggaaca catgtaagcg acggatgtgg 32220

caaaagtgac gtttttggtg tgcgccggtg tacacaggaa gtgacaattt tcgcgcggtt 32280

ttaggcggat gttgtagtaa atttgggcgt aaccgagtaa gatttggcca ttttcgcggg 32340

aaaactgaat aagaggaagt gaaatctgaa taattttgtg ttactcatag cgcgtaatct 32400

ctagcat 32407

<210> 2

<211> 6663

<212> DNA

<213> adenovirus

<400> 2

cgtaactata acggtcctaa ggtagcgaaa gctcagatct cccgatcccc tatggtgcac 60

tctcagtaca atctgctctg atgccgcata gttaagccag tatctgctcc ctgcttgtgt 120

gttggaggtc gctgagtagt gcgcgagcaa aatttaagct acaacaaggc aaggcttgac 180

cgacaattga gtactggatc cgatatcgtc gacgctagcg tttaaacggg ccctctagac 240

tcgagccgat gtcgaggatc cggactgaaa atgagacata ttatctgcca cggaggtgtt 300

attaccgaag aaatggccgc cagtcttttg gaccagctga tcgaagaggt actggctgat 360

aatcttccac ctcctagcca ttttgaacca cctacccttc acgaactgta tgatttagac 420

gtgacggccc ccgaagatcc caacgaggag gcggtttcgc agatttttcc cgactctgta 480

atgttggcgg tgcaggaagg gattgactta ctcacttttc cgccggcgcc cggttctccg 540

gagccgcctc acctttcccg gcagcccgag cagccggagc agagagcctt gggtccggtt 600

tctatgccaa accttgtacc ggaggtgatc gatcttacct gccacgaggc tggctttcca 660

cccagtgacg acgaggatga agagggtgag gagtttgtgt tagattatgt ggagcacccc 720

gggcacggtt gcaggtcttg tcattatcac cggaggaata cgggggaccc agatattatg 780

tgttcgcttt gctatatgag gacctgtggc atgtttgtct acagtaagtg aaaattatgg 840

gcagtgggtg atagagtggt gggtttggtg tggtaatttt ttttttaatt tttacagttt 900

tgtggtttaa agaattttgt attgtgattt ttttaaaagg tcctgtgtct gaacctgagc 960

ctgagcccga gccagaaccg gagcctgcaa gacctacccg ccgtcctaaa atggcgcctg 1020

ctatcctgag acgcccgaca tcacctgtgt ctagagaatg caatagtagt acggatagct 1080

gtgactccgg tccttctaac acacctcctg agatacaccc ggtggtcccg ctgtgcccca 1140

ttaaaccagt tgccgtgaga gttggtgggc gtcgccaggc tgtggaatgt atcgaggact 1200

tgcttaacga gcctgggcaa cctttggact tgagctgtaa acgccccagg ccataaggtg 1260

taaacctgtg attgcgtgtg tggttaacgc ctttgtttgc tgaatgagtt gatgtaagtt 1320

taataaaggg tgagataatg tttaacttgc atggcgtgtt aaatggggcg gggcttaaag 1380

ggtatataat gcgccgtggg ctaatcttgg ttacatctga cctcatggag gcttgggagt 1440

gtttggaaga tttttctgct gtgcgtaact tgctggaaca gagctctaac agtacctctt 1500

ggttttggag gtttctgtgg ggctcatccc aggcaaagtt agtctgcaga attaaggagg 1560

attacaagtg ggaatttgaa gagcttttga aatcctgtgg tgagctgttt gattctttga 1620

atctgggtca ccaggcgctt ttccaagaga aggtcatcaa gactttggat ttttccacac 1680

cggggcgcgc tgcggctgct gttgcttttt tgagttttat aaaggataaa tggagcgaag 1740

aaacccatct gagcgggggg tacctgctgg attttctggc catgcatctg tggagagcgg 1800

ttgtgagaca caagaatcgc ctgctactgt tgtcttccgt ccgcccggcg ataataccga 1860

cggaggagca gcagcagcag caggaggaag ccaggcggcg gcggcaggag cagagcccat 1920

ggaacccgag agccggcctg gaccctcggg aatgaatgtt gtacaggtgg ctgaactgta 1980

tccagaactg agacgcattt tgacaattac agaggatggg caggggctaa agggggtaaa 2040

gagggagcgg ggggcttgtg aggctacaga ggaggctagg aatctagctt ttagcttaat 2100

gaccagacac cgtcctgagt gtattacttt tcaacagatc aaggataatt gcgctaatga 2160

gcttgatctg ctggcgcaga agtattccat agagcagctg accacttact ggctgcagcc 2220

aggggatgat tttgaggagg ctattagggt atatgcaaag gtggcactta ggccagattg 2280

caagtacaag atcagcaaac ttgtaaatat caggaattgt tgctacattt ctgggaacgg 2340

ggccgaggtg gagatagata cggaggatag ggtggccttt agatgtagca tgataaatat 2400

gtggccgggg gtgcttggca tggacggggt ggttattatg aatgtaaggt ttactggccc 2460

caattttagc ggtacggttt tcctggccaa taccaacctt atcctacacg gtgtaagctt 2520

ctatgggttt aacaatacct gtgtggaagc ctggaccgat gtaaggttcc ggggctgtgc 2580

cttttactgc tgctggaagg gggtggtgtg tcgccccaaa agcagggctt caattaagaa 2640

atgcctcttt gaaaggtgta ccttgggtat cctgtctgag ggtaactcca gggtgcgcca 2700

caatgtggcc tccgactgtg gttgcttcat gctagtgaaa agcgtggctg tgattaagca 2760

taacatggta tgtggcaact gcgaggacag ggcctctcag atgctgacct gctcggacgg 2820

caactgtcac ctgctgaaga ccattcacgt agccagccac tctcgcaagg cctggccagt 2880

gtttgagcat aacatactga cccgctgttc cttgcatttg ggtaacagga ggggggtgtt 2940

cctaccttac caatgcaatt tgagtcacac taagatattg cttgagcccg agagcatgtc 3000

caaggtgaac ctgaacgggg tgtttgacat gaccatgaag atctggaagg tgctgaggta 3060

cgatgagacc cgcaccaggt gcagaccctg cgagtgtggc ggtaaacata ttaggaacca 3120

gcctgtgatg ctggatgtga ccgaggagct gaggcccgat cacttggtgc tggcctgcac 3180

ccgcgctgag tttggctcta gcgatgaaga tacagattga ggtactgaaa tgtgtgggcg 3240

tggcttaagg gtgggaaaga atatataagg tgggggtctt atgtagtttt gtatctgttt 3300

tgcagcagcc gccgccgcca tgagcaccaa ctcgtttgat ggaagcattg tgagctcata 3360

tttgacaacg cgcatgcccc catgggccgg ggtgcgtcag aatgtgatgg gctccagcat 3420

tgatggtcgc cccgtcctgc ccgcaaactc tactaccttg acctacgaga ccgtgtctgg 3480

aacgccgttg gagactgcag cctccgccgc cgcttcagcc gctgcagcca ccgcccgcgg 3540

gattgtgact gactttgctt tcctgagccc gcttgcaagc agtgcagctt cccgttcatc 3600

cgcccgcgat gacaagttga cggctctttt ggcacaattg gccgccactg tgctggatga 3660

tccgagctcg gtaccaagct taagtaagtg actagaactt gtttattgca gcttataatg 3720

gttacaaata aagcaatagc atcacaaatt tcacaaataa agcatttttt tcactgcatt 3780

ctagttgtgg tttgtccaaa ctcatctgca gatctgaatt catctatgtc gggtgcggag 3840

aaagaggtaa tgaaatggca ttatgggtat tatgggtctg cattaatgaa tcggccaacg 3900

cgcggggaga ggcggtttgc gtattgggcg ctcttccgct tcctcgctca ctgactcgct 3960

gcgctcggtc gttcggctgc ggcgagcggt atcagctcac tcaaaggcgg taatacggtt 4020

atccacagaa tcaggggata acgcaggaaa gaacatgtga gcaaaaggcc agcaaaaggc 4080

caggaaccgt aaaaaggccg cgttgctggc gtttttccat aggctccgcc cccctgacga 4140

gcatcacaaa aatcgacgct caagtcagag gtggcgaaac ccgacaggac tataaagata 4200

ccaggcgttt ccccctggaa gctccctcgt gcgctctcct gttccgaccc tgccgcttac 4260

cggatacctg tccgcctttc tcccttcggg aagcgtggcg ctttctcaat gctcacgctg 4320

taggtatctc agttcggtgt aggtcgttcg ctccaagctg ggctgtgtgc acgaaccccc 4380

cgttcagccc gaccgctgcg ccttatccgg taactatcgt cttgagtcca acccggtaag 4440

acacgactta tcgccactgg cagcagccac tggtaacagg attagcagag cgaggtatgt 4500

aggcggtgct acagagttct tgaagtggtg gcctaactac ggctacacta gaaggacagt 4560

atttggtatc tgcgctctgc tgaagccagt taccttcgga aaaagagttg gtagctcttg 4620

atccggcaaa caaaccaccg ctggtagcgg tggttttttt gtttgcaagc agcagattac 4680

gcgcagaaaa aaaggatctc aagaagatcc tttgatcttt tctacggggt ctgacgctca 4740

gtggaacgaa aactcacgtt aagggatttt ggtcatgaga ttatcaaaaa ggatcttcac 4800

ctagatcctt ttgatcctcc ggcgttcagc ctgtgccaca gccgacagga tggtgaccac 4860

catttgcccc atatcaccgt cggtactgat cccgtcgtca ataaaccgaa ccgctacacc 4920

ctgagcatca aactctttta tcagttggat catgtcggcg gtgtcgcggc caagacggtc 4980

gagcttcttc accagaatga catcaccttc ctccaccttc atcctcagca aatccagccc 5040

ttcccgatct gttgaactgc cggatgcctt gtcggtaaag atgcggttag cttttacccc 5100

tgcatctttg agcgctgagg tctgcctcgt gaagaaggtg ttgctgactc ataccaggcc 5160

tgaatcgccc catcatccag ccagaaagtg agggagccac ggttgatgag agctttgttg 5220

taggtggacc agttggtgat tttgaacttt tgctttgcca cggaacggtc tgcgttgtcg 5280

ggaagatgcg tgatctgatc cttcaactca gcaaaagttc gatttattca acaaagccgc 5340

cgtcccgtca agtcagcgta atgctctgcc agtgttacaa ccaattaacc aattctgatt 5400

agaaaaactc atcgagcatc aaatgaaact gcaatttatt catatcagga ttatcaatac 5460

catatttttg aaaaagccgt ttctgtaatg aaggagaaaa ctcaccgagg cagttccata 5520

ggatggcaag atcctggtat cggtctgcga ttccgactcg tccaacatca atacaaccta 5580

ttaatttccc ctcgtcaaaa ataaggttat caagtgagaa atcaccatga gtgacgactg 5640

aatccggtga gaatggcaaa agcttatgca tttctttcca gacttgttca acaggccagc 5700

cattacgctc gtcatcaaaa tcactcgcat caaccaaacc gttattcatt cgtgattgcg 5760

cctgagcgag acgaaatacg cgatcgctgt taaaaggaca attacaaaca ggaatcgaat 5820

gcaaccggcg caggaacact gccagcgcat caacaatatt ttcacctgaa tcaggatatt 5880

cttctaatac ctggaatgct gttttcccgg ggatcgcagt ggtgagtaac catgcatcat 5940

caggagtacg gataaaatgc ttgatggtcg gaagaggcat aaattccgtc agccagttta 6000

gtctgaccat ctcatctgta acatcattgg caacgctacc tttgccatgt ttcagaaaca 6060

actctggcgc atcgggcttc ccatacaatc gatagattgt cgcacctgat tgcccgacat 6120
tatcgcgagc ccatttatac ccatataaat cagcatccat gttggaattt aatcgcggcc 6180
tcgagcaaga cgtttcccgt tgaatatggc tcataacacc ccttgtatta ctgtttatgt 6240
aagcagacag ttttattgtt catgatgata tatttttatc ttgtgcaatg taacatcaga 6300
gattttgaga cacaacgtgg ctttgttgaa taaatcgaac ttttgctgag ttgaaggatc 6360
agatcacgca tcttcccgac aacgcagacc gttccgtggc aaagcaaaag ttcaaaatca 6420
ccaactggtc cacctacaac aaagctctca tcaaccgtgg ctccctcact ttctggctgg 6480
atgatggggc gattcaggcc tggtatgagt cagcaacacc ttcttcacga ggcagacctc 6540
agcgctagat tattgaagca tttatcaggg ttattgtctc atgagcggat acatatttga 6600
atgtatttag aaaaataaac aaataggggt tccgcgcaca tttccccgaa aagtgccacc 6660
tga 6663

<210> 3

<211> 36187

<212> DNA

<213> adenovirus

<400> 3

cgtaactata acggtcctaa ggtagcgaaa gctcagatct cccgatcccc tatggtgcac 60
tctcagtaca atctgctctg atgccgcata gttaagccag tatctgctcc ctgcttgtgt 120
gttggaggtc gctgagtagt gcgcgagcaa aatttaagct acaacaaggc aaggcttgac 180
cgacaattga gtactggatc cgatatcgtc gacgctagcg tttaaacggg ccctctagac 240
tcgagccgat gtcgaggatc cggactgaaa atgagacata ttatctgcca cggaggtgtt 300
attaccgaag aaatggccgc cagtcttttg gaccagctga tcgaagaggt actggctgat 360
aatcttccac ctcctagcca ttttgaacca cctacccttc acgaactgta tgatttagac 420
gtgacggccc ccgaagatcc caacgaggag gcggtttcgc agatttttcc cgactctgta 480
atgttggcgg tgcaggaagg gattgactta ctcacttttc cgccggcgcc cggttctccg 540
gagccgcctc acctttcccg gcagcccgag cagccggagc agagagcctt gggtccggtt 600
tctatgccaa accttgtacc ggaggtgatc gatcttacct gccacgaggc tggctttcca 660
cccagtgacg acgaggatga agagggtgag gagtttgtgt tagattatgt ggagcacccc 720
gggcacggtt gcaggtcttg tcattatcac cggaggaata cgggggaccc agatattatg 780
tgttcgcttt gctatatgag gacctgtggc atgtttgtct acagtaagtg aaaattatgg 840
gcagtgggtg atagagtggt gggtttggtg tggtaatttt ttttttaatt tttacagttt 900
tgtggtttaa agaattttgt attgtgattt ttttaaaagg tcctgtgtct gaacctgagc 960

ctgagcccga gccagaaccg gagcctgcaa gacctacccg ccgtcctaaa atggcgcctg 1020

ctatcctgag acgcccgaca tcacctgtgt ctagagaatg caatagtagt acggatagct 1080

gtgactccgg tccttctaac acacctcctg agatacaccc ggtggtcccg ctgtgcccca 1140

ttaaaccagt tgccgtgaga gttggtgggc gtcgccaggc tgtggaatgt atcgaggact 1200

tgcttaacga gcctgggcaa cctttggact tgagctgtaa acgccccagg ccataaggtg 1260

taaacctgtg attgcgtgtg tggttaacgc ctttgtttgc tgaatgagtt gatgtaagtt 1320

taataaaggg tgagataatg tttaacttgc atggcgtgtt aaatggggcg gggcttaaag 1380

ggtatataat gcgccgtggg ctaatcttgg ttacatctga cctcatggag gcttgggagt 1440

gtttggaaga tttttctgct gtgcgtaact tgctggaaca gagctctaac agtacctctt 1500

ggttttggag gtttctgtgg ggctcatccc aggcaaagtt agtctgcaga attaaggagg 1560

attacaagtg ggaatttgaa gagcttttga aatcctgtgg tgagctgttt gattctttga 1620

atctgggtca ccaggcgctt ttccaagaga aggtcatcaa gactttggat ttttccacac 1680

cggggcgcgc tgcggctgct gttgcttttt tgagttttat aaaggataaa tggagcgaag 1740

aaacccatct gagcgggggg tacctgctgg attttctggc catgcatctg tggagagcgg 1800

ttgtgagaca caagaatcgc ctgctactgt tgtcttccgt ccgcccggcg ataataccga 1860

cggaggagca gcagcagcag caggaggaag ccaggcggcg gcggcaggag cagagcccat 1920

ggaacccgag agccggcctg gaccctcggg aatgaatgtt gtacaggtgg ctgaactgta 1980

tccagaactg agacgcattt tgacaattac agaggatggg caggggctaa agggggtaaa 2040

gagggagcgg ggggcttgtg aggctacaga ggaggctagg aatctagctt ttagcttaat 2100

gaccagacac cgtcctgagt gtattacttt tcaacagatc aaggataatt gcgctaatga 2160

gcttgatctg ctggcgcaga agtattccat agagcagctg accacttact ggctgcagcc 2220

aggggatgat tttgaggagg ctattagggt atatgcaaag gtggcactta ggccagattg 2280

caagtacaag atcagcaaac ttgtaaatat caggaattgt tgctacattt ctgggaacgg 2340

ggccgaggtg gagatagata cggaggatag ggtggccttt agatgtagca tgataaatat 2400

gtggccgggg gtgcttggca tggacggggt ggttattatg aatgtaaggt ttactggccc 2460

caattttagc ggtacggttt tcctggccaa taccaacctt atcctacacg gtgtaagctt 2520

ctatgggttt aacaatacct gtgtggaagc ctggaccgat gtaaggttcc ggggctgtgc 2580

cttttactgc tgctggaagg gggtggtgtg tcgccccaaa agcagggctt caattaagaa 2640

atgcctcttt gaaaggtgta ccttgggtat cctgtctgag ggtaactcca gggtgcgcca 2700

caatgtggcc tccgactgtg gttgcttcat gctagtgaaa agcgtggctg tgattaagca 2760

taacatggta tgtggcaact gcgaggacag ggcctctcag atgctgacct gctcggacgg 2820

caactgtcac ctgctgaaga ccattcacgt agccagccac tctcgcaagg cctggccagt 2880

gtttgagcat aacatactga cccgctgttc cttgcatttg ggtaacagga ggggggtgtt 2940

cctaccttac caatgcaatt tgagtcacac taagatattg cttgagcccg agagcatgtc 3000

caaggtgaac ctgaacgggg tgtttgacat gaccatgaag atctggaagg tgctgaggta 3060

cgatgagacc cgcaccaggt gcagaccctg cgagtgtggc ggtaaacata ttaggaacca 3120

gcctgtgatg ctggatgtga ccgaggagct gaggcccgat cacttggtgc tggcctgcac 3180

ccgcgctgag tttggctcta gcgatgaaga tacagattga ggtactgaaa tgtgtgggcg 3240

tggcttaagg gtgggaaaga atatataagg tgggggtctt atgtagtttt gtatctgttt 3300

tgcagcagcc gccgccgcca tgagcaccaa ctcgtttgat ggaagcattg tgagctcata 3360

tttgacaacg cgcatgcccc catgggccgg ggtgcgtcag aatgtgatgg gctccagcat 3420

tgatggtcgc cccgtcctgc ccgcaaactc tactaccttg acctacgaga ccgtgtctgg 3480

aacgccgttg gagactgcag cctccgccgc cgcttcagcc gctgcagcca ccgcccgcgg 3540

gattgtgact gactttgctt tcctgagccc gcttgcaagc agtgcagctt cccgttcatc 3600

cgcccgcgat gacaagttga cggctctttt ggcacaattg gccgccactg tgctggatga 3660

tccgagctcg gtaccaagct taagtaagtg actagaactt gtttattgca gcttataatg 3720

gttacaaata aagcaatagc atcacaaatt tcacaaataa agcatttttt tcactgcatt 3780

ctagttgtgg tttgtccaaa ctcatctgca gatctgaatt catctatgtc gggtgcggag 3840

aaagaggtaa tgaaatggca tcgactcgaa gatctgggcg tggttaaggg tgggaaagaa 3900

tatataaggt gggggtctta tgtagttttg tatctgtttt gcagcagccg ccgccgccat 3960

gagcaccaac tcgtttgatg gaagcattgt gagctcatat ttgacaacgc gcatgccccc 4020

atgggccggg gtgcgtcaga atgtgatggg ctccagcatt gatggtcgcc ccgtcctgcc 4080

cgcaaactct actaccttga cctacgagac cgtgtctgga acgccgttgg agactgcagc 4140

ctccgccgcc gcttcagccg ctgcagccac cgcccgcggg attgtgactg actttgcttt 4200

cctgagcccg cttgcaagca gtgcagcttc ccgttcatcc gcccgcgatg acaagttgac 4260

ggctcttttg gcacaattgg attctttgac ccgggaactt aatgtcgttt ctcagcagct 4320

gttggatctg cgccagcagg tttctgccct gaaggcttcc tcccctccca atgcggttta 4380

aaacataaat aaaaaaccag actctgtttg gatttggatc aagcaagtgt cttgctgtct 4440

ttatttaggg gttttgcgcg cgcggtaggc ccgggaccag cggtctcggt cgttgagggt 4500

cctgtgtatt ttttccagga cgtggtaaag gtgactctgg atgttcagat acatgggcat 4560

aagcccgtct ctggggtgga ggtagcacca ctgcagagct tcatgctgcg gggtggtgtt 4620

gtagatgatc cagtcgtagc aggagcgctg ggcgtggtgc ctaaaaatgt ctttcagtag 4680

caagctgatt gccaggggca ggcccttggt gtaagtgttt acaaagcggt taagctggga 4740

tgggtgcata cgtggggata tgagatgcat cttggactgt atttttaggt tggctatgtt 4800

cccagccata tccctccggg gattcatgtt gtgcagaacc accagcacag tgtatccggt 4860

gcacttggga aatttgtcat gtagcttaga aggaaatgcg tggaagaact tggagacgcc 4920

cttgtgacct ccaagatttt ccatgcattc gtccataatg atggcaatgg gcccacgggc 4980

ggcggcctgg gcgaagatat ttctgggatc actaacgtca tagttgtgtt ccaggatgag 5040

atcgtcatag gccattttta caaagcgcgg gcggagggtg ccagactgcg gtataatggt 5100

tccatccggc ccaggggcgt agttaccctc acagatttgc atttcccacg ctttgagttc 5160

agatgggggg atcatgtcta cctgcggggc gatgaagaaa acggtttccg gggtagggga 5220

gatcagctgg gaagaaagca ggttcctgag cagctgcgac ttaccgcagc cggtgggccc 5280

gtaaatcaca cctattaccg gctgcaactg gtagttaaga gagctgcagc tgccgtcatc 5340

cctgagcagg ggggccactt cgttaagcat gtccctgact cgcatgtttt ccctgaccaa 5400

atccgccaga aggcgctcgc cgcccagcga tagcagttct tgcaaggaag caaagttttt 5460

caacggtttg agaccgtccg ccgtaggcat gcttttgagc gtttgaccaa gcagttccag 5520

gcggtcccac agctcggtca cctgctctac ggcatctcga tccagcatat ctcctcgttt 5580

cgcgggttgg ggcggctttc gctgtacggc agtagtcggt gctcgtccag acgggccagg 5640

gtcatgtctt tccacgggcg cagggtcctc gtcagcgtag tctgggtcac ggtgaagggg 5700

tgcgctccgg gctgcgcgct ggccagggtg cgcttgaggc tggtcctgct ggtgctgaag 5760

cgctgccggt cttcgccctg cgcgtcggcc aggtagcatt tgaccatggt gtcatagtcc 5820

agcccctccg cggcgtggcc cttggcgcgc agcttgccct tggaggaggc gccgcacgag 5880

gggcagtgca gacttttgag ggcgtagagc ttgggcgcga gaaataccga ttccggggag 5940

taggcatccg cgccgcaggc cccgcagacg gtctcgcatt ccacgagcca ggtgagctct 6000

ggccgttcgg ggtcaaaaac caggtttccc ccatgctttt tgatgcgttt cttacctctg 6060

gtttccatga gccggtgtcc acgctcggtg acgaaaaggc tgtccgtgtc cccgtataca 6120

gacttgagag gcctgtcctc gagcggtgtt ccgcggtcct cctcgtatag aaactcggac 6180

cactctgaga caaaggctcg cgtccaggcc agcacgaagg aggctaagtg ggaggggtag 6240

cggtcgttgt ccactagggg gtccactcgc tccagggtgt gaagacacat gtcgccctct 6300

tcggcatcaa ggaaggtgat tggtttgtag gtgtaggcca cgtgaccggg tgttcctgaa 6360

ggggggctat aaaagggggt gggggcgcgt tcgtcctcac tctcttccgc atcgctgtct 6420

gcgagggcca gctgttgggg tgagtactcc ctctgaaaag cgggcatgac ttctgcgcta 6480

agattgtcag tttccaaaaa cgaggaggat ttgatattca cctggcccgc ggtgatgcct 6540

ttgagggtgg ccgcatccat ctggtcagaa aagacaatct ttttgttgtc aagcttggtg 6600

gcaaacgacc cgtagagggc gttggacagc aacttggcga tggagcgcag ggtttggttt 6660

ttgtcgcgat cggcgcgctc cttggccgcg atgtttagct gcacgtattc gcgcgcaacg 6720

caccgccatt cgggaaagac ggtggtgcgc tcgtcgggca ccaggtgcac gcgccaaccg 6780

cggttgtgca gggtgacaag gtcaacgctg gtggctacct ctccgcgtag gcgctcgttg 6840

gtccagcaga ggcggccgcc cttgcgcgag cagaatggcg gtagggggtc tagctgcgtc 6900

tcgtccgggg ggtctgcgtc cacggtaaag accccgggca gcaggcgcgc gtcgaagtag 6960

tctatcttgc atccttgcaa gtctagcgcc tgctgccatg cgcgggcggc aagcgcgcgc 7020

tcgtatgggt tgagtggggg accccatggc atggggtggg tgagcgcgga ggcgtacatg 7080

ccgcaaatgt cgtaaacgta gaggggctct ctgagtattc caagatatgt agggtagcat 7140

cttccaccgc ggatgctggc gcgcacgtaa tcgtatagtt cgtgcgaggg agcgaggagg 7200

tcgggaccga ggttgctacg ggcgggctgc tctgctcgga agactatctg cctgaagatg 7260

gcatgtgagt tggatgatat ggttggacgc tggaagacgt tgaagctggc gtctgtgaga 7320

cctaccgcgt cacgcacgaa ggaggcgtag gagtcgcgca gcttgttgac cagctcggcg 7380

gtgacctgca cgtctagggc gcagtagtcc agggtttcct tgatgatgtc atacttatcc 7440

tgtccctttt ttttccacag ctcgcggttg aggacaaact cttcgcggtc tttccagtac 7500

tcttggatcg gaaacccgtc ggcctccgaa cggtaagagc ctagcatgta gaactggttg 7560

acggcctggt aggcgcagca tcccttttct acgggtagcg cgtatgcctg cgcggccttc 7620

cggagcgagg tgtgggtgag cgcaaaggtg tccctgacca tgactttgag gtactggtat 7680

ttgaagtcag tgtcgtcgca tccgccctgc tcccagagca aaaagtccgt gcgctttttg 7740

gaacgcggat ttggcagggc gaaggtgaca tcgttgaaga gtatctttcc cgcgcgaggc 7800

ataaagttgc gtgtgatgcg gaagggtccc ggcacctcgg aacggttgtt aattacctgg 7860

gcggcgagca cgatctcgtc aaagccgttg atgttgtggc ccacaatgta aagttccaag 7920

aagcgcggga tgcccttgat ggaaggcaat tttttaagtt cctcgtaggt gagctcttca 7980

ggggagctga gcccgtgctc tgaaagggcc cagtctgcaa gatgagggtt ggaagcgacg 8040

aatgagctcc acaggtcacg ggccattagc atttgcaggt ggtcgcgaaa ggtcctaaac 8100

tggcgaccta tggccatttt ttctggggtg atgcagtaga aggtaagcgg gtcttgttcc 8160

cagcggtccc atccaaggtt cgcggctagg tctcgcgcgg cagtcactag aggctcatct 8220

ccgccgaact tcatgaccag catgaagggc acgagctgct tcccaaaggc ccccatccaa 8280

gtataggtct ctacatcgta ggtgacaaag agacgctcgg tgcgaggatg cgagccgatc 8340

gggaagaact ggatctcccg ccaccaattg gaggagtggc tattgatgtg gtgaaagtag 8400

aagtccctgc gacgggccga acactcgtgc tggcttttgt aaaaacgtgc gcagtactgg 8460

cagcggtgca cgggctgtac atcctgcacg aggttgacct gacgaccgcg cacaaggaag 8520

cagagtggga atttgagccc ctcgcctggc gggtttggct ggtggtcttc tacttcggct 8580

gcttgtcctt gaccgtctgg ctgctcgagg ggagttacgg tggatcggac caccacgccg 8640

cgcgagccca aagtccagat gtccgcgcgc ggcggtcgga gcttgatgac aacatcgcgc 8700

agatgggagc tgtccatggt ctggagctcc cgcggcgtca ggtcaggcgg gagctcctgc 8760

aggtttacct cgcatagacg ggtcagggcg cgggctagat ccaggtgata cctaatttcc 8820

aggggctggt tggtggcggc gtcgatggct tgcaagaggc cgcatccccg cggcgcgact 8880

acggtaccgc gcggcgggcg gtgggccgcg ggggtgtcct tggatgatgc atctaaaagc 8940
ggtgacgcgg gcgagccccc ggaggtaggg ggggctccgg acccgccggg agagggggca 9000
ggggcacgtc ggcgccgcgc gcgggcagga gctggtgctg cgcgcgtagg ttgctggcga 9060
acgcgacgac gcggcggttg atctcctgaa tctggcgcct ctgcgtgaag acgacgggcc 9120
cggtgagctt gaacctgaaa gagagttcga cagaatcaat ttcggtgtcg ttgacggcgg 9180
cctggcgcaa aatctcctgc acgtctcctg agttgtcttg ataggcgatc tcggccatga 9240
actgctcgat ctcttcctcc tggagatctc cgcgtccggc tcgctccacg gtggcggcga 9300
ggtcgttgga aatgcgggcc atgagctgcg agaaggcgtt gaggcctccc tcgttccaga 9360
cgcggctgta gaccacgccc ccttcggcat cgcgggcgcg catgaccacc tgcgcgagat 9420
tgagctccac gtgccgggcg aagacggcgt agtttcgcag gcgctgaaag aggtagttga 9480
gggtggtggc ggtgtgttct gccacgaaga agtacataac ccagcgtcgc aacgtggatt 9540
cgttgatatc ccccaaggcc tcaaggcgct ccatggcctc gtagaagtcc acggcgaagt 9600
tgaaaaactg ggagttgcgc gccgacacgg ttaactcctc ctccagaaga cggatgagct 9660
cggcgacagt gtcgcgcacc tcgcgctcaa aggctacagg ggcctcttct tcttcttcaa 9720
tctcctcttc cataagggcc tccccttctt cttcttctgg cggcggtggg ggagggggga 9780
cacggcggcg acgacggcgc accgggaggc ggtcgacaaa gcgctcgatc atctccccgc 9840
ggcgacggcg catggtctcg gtgacggcgc ggccgttctc gcgggggcgc agttggaaga 9900
cgccgcccgt catgtcccgg ttatgggttg gcggggggct gccatgcggc agggatacgg 9960
cgctaacgat gcatctcaac aattgttgtg taggtactcc gccgccgagg gacctgagcg 10020
agtccgcatc gaccggatcg gaaaacctct cgagaaaggc gtctaaccag tcacagtcgc 10080
aaggtaggct gagcaccgtg gcgggcggca gcgggcggcg gtcggggttg tttctggcgg 10140
aggtgctgct gatgatgtaa ttaaagtagg cggtcttgag acggcggatg gtcgacagaa 10200
gcaccatgtc cttgggtccg gcctgctgaa tgcgcaggcg gtcggccatg ccccaggctt 10260
cgttttgaca tcggcgcagg tctttgtagt agtcttgcat gagcctttct accggcactt 10320
cttcttctcc ttcctcttgt cctgcatctc ttgcatctat cgctgcggcg gcggcggagt 10380
ttggccgtag gtggcgccct cttcctccca tgcgtgtgac cccgaagccc ctcatcggct 10440
gaagcagggc taggtcggcg acaacgcgct cggctaatat ggcctgctgc acctgcgtga 10500
gggtagactg gaagtcatcc atgtccacaa agcggtggta tgcgcccgtg ttgatggtgt 10560
aagtgcagtt ggccataacg gaccagttaa cggtctggtg acccggctgc gagagctcgg 10620
tgtacctgag acgcgagtaa gccctcgagt caaatacgta gtcgttgcaa gtccgcacca 10680
ggtactggta tcccaccaaa aagtgcggcg gcggctggcg gtagaggggc cagcgtaggg 10740
tggccggggc tccgggggcg agatcttcca acataaggcg atgatatccg tagatgtacc 10800
tggacatcca ggtgatgccg gcggcggtgg tggaggcgcg cggaaagtcg cggacgcggt 10860

tccagatgtt gcgcagcggc aaaaagtgct ccatggtcgg gacgctctgg ccggtcaggc 10920

gcgcgcaatc gttgacgctc tagcgtgcaa aaggagagcc tgtaagcggg cactcttccg 10980

tggtctggtg gataaattcg caagggtatc atggcggacg accggggttc gagccccgta 11040

tccggccgtc cgccgtgatc catgcggtta ccgcccgcgt gtcgaaccca ggtgtgcgac 11100

gtcagacaac gggggagtgc tccttttggc ttccttccag gcgcggcggc tgctgcgcta 11160

gcttttttgg ccactggccg cgcgcagcgt aagcggttag gctggaaagc gaaagcatta 11220

agtggctcgc tccctgtagc cggagggtta ttttccaagg gttgagtcgc gggacccccg 11280

gttcgagtct cggaccggcc ggactgcggc gaacgggggt ttgcctcccc gtcatgcaag 11340

accccgcttg caaattcctc cggaaacagg gacgagcccc ttttttgctt ttcccagatg 11400

catccggtgc tgcggcagat gcgcccccct cctcagcagc ggcaagagca agagcagcgg 11460

cagacatgca gggcaccctc ccctcctcct accgcgtcag gaggggcgac atccgcggtt 11520

gacgcggcag cagatggtga ttacgaaccc ccgcggcgcc gggcccggca ctacctggac 11580

ttggaggagg gcgagggcct ggcgcggcta ggagcgccct ctcctgagcg gcacccaagg 11640

gtgcagctga agcgtgatac gcgtgaggcg tacgtgccgc ggcagaacct gtttcgcgac 11700

cgcgagggag aggagcccga ggagatgcgg gatcgaaagt tccacgcagg gcgcgagctg 11760

cggcatggcc tgaatcgcga gcggttgctg cgcgaggagg actttgagcc cgacgcgcga 11820

accgggatta gtcccgcgcg cgcacacgtg gcggccgccg acctggtaac cgcatacgag 11880

cagacggtga accaggagat taactttcaa aaaagcttta acaaccacgt gcgtacgctt 11940

gtggcgcgcg aggaggtggc tataggactg atgcatctgt gggactttgt aagcgcgctg 12000

gagcaaaacc caaatagcaa gccgctcatg gcgcagctgt tccttatagt gcagcacagc 12060

agggacaacg aggcattcag ggatgcgctg ctaaacatag tagagcccga gggccgctgg 12120

ctgctcgatt tgataaacat cctgcagagc atagtggtgc aggagcgcag cttgagcctg 12180

gctgacaagg tggccgccat caactattcc atgcttagcc tgggcaagtt ttacgcccgc 12240

aagatatacc atacccctta cgttcccata gacaaggagg taaagatcga ggggttctac 12300

atgcgcatgg cgctgaaggt gcttaccttg agcgacgacc tgggcgttta tcgcaacgag 12360

cgcatccaca aggccgtgag cgtgagccgg cggcgcgagc tcagcgaccg cgagctgatg 12420

cacagcctgc aaagggccct ggctggcacg ggcagcggcg atagagaggc cgagtcctac 12480

tttgacgcgg gcgctgacct gcgctgggcc ccaagccgac gcgccctgga ggcagctggg 12540

gccggacctg ggctggcggt ggcacccgcg cgcgctggca acgtcggcgg cgtggaggaa 12600

tatgacgagg acgatgagta cgagccagag gacggcgagt actaagcggt gatgtttctg 12660

atcagatgat gcaagacgca acggacccgg cggtgcgggc ggcgctgcag agccagccgt 12720

ccggccttaa ctccacggac gactggcgcc aggtcatgga ccgcatcatg tcgctgactg 12780

cgcgcaatcc tgacgcgttc cggcagcagc cgcaggccaa ccggctctcc gcaattctgg 12840

aagcggtggt cccggcgcgc gcaaacccca cgcacgagaa ggtgctggcg atcgtaaacg 12900
cgctggccga aaacagggcc atccggcccg acgaggccgg cctggtctac gacgcgctgc 12960
ttcagcgcgt ggctcgttac aacagcggca acgtgcagac caacctggac cggctggtgg 13020
gggatgtgcg cgaggccgtg gcgcagcgtg agcgcgcgca gcagcagggc aacctgggct 13080
ccatggttgc actaaacgcc ttcctgagta cacagcccgc caacgtgccg cggggacagg 13140
aggactacac caactttgtg agcgcactgc ggctaatggt gactgagaca ccgcaaagtg 13200
aggtgtacca gtctgggcca gactattttt tccagaccag tagacaaggc ctgcagaccg 13260
taaacctgag ccaggctttc aaaaacttgc aggggctgtg gggggtgcgg gctcccacag 13320
gcgaccgcgc gaccgtgtct agcttgctga cgcccaactc gcgcctgttg ctgctgctaa 13380
tagcgccctt cacggacagt ggcagcgtgt cccgggacac atacctaggt cacttgctga 13440
cactgtaccg cgaggccata ggtcaggcgc atgtggacga gcatactttc caggagatta 13500
caagtgtcag ccgcgcgctg gggcaggagg acacgggcag cctggaggca accctaaact 13560
acctgctgac caaccggcgg cagaagatcc cctcgttgca cagtttaaac agcgaggagg 13620
agcgcatttt gcgctacgtg cagcagagcg tgagccttaa cctgatgcgc gacggggtaa 13680
cgcccagcgt ggcgctggac atgaccgcgc gcaacatgga accgggcatg tatgcctcaa 13740
accggccgtt tatcaaccgc ctaatggact acttgcatcg cgcggccgcc gtgaaccccg 13800
agtatttcac caatgccatc ttgaacccgc actggctacc gccccctggt ttctacaccg 13860
ggggattcga ggtgcccgag ggtaacgatg gattcctctg ggacgacata gacgacagcg 13920
tgttttcccc gcaaccgcag accctgctag agttgcaaca gcgcgagcag gcagaggcgg 13980
cgctgcgaaa ggaaagcttc cgcaggccaa gcagcttgtc cgatctaggc gctgcggccc 14040
cgcggtcaga tgctagtagc ccatttccaa gcttgatagg gtctcttacc agcactcgca 14100
ccacccgccc gcgcctgctg ggcgaggagg agtacctaaa caactcgctg ctgcagccgc 14160
agcgcgaaaa aaacctgcct ccggcatttc ccaacaacgg gatagagagc ctagtggaca 14220
agatgagtag atggaagacg tacgcgcagg agcacaggga cgtgccaggc ccgcgcccgc 14280
ccacccgtcg tcaaaggcac gaccgtcagc ggggtctggt gtgggaggac gatgactcgg 14340
cagacgacag cagcgtcctg gatttgggag ggagtggcaa cccgtttgcg caccttcgcc 14400
ccaggctggg gagaatgttt taaaaaaaaa aaaagcatga tgcaaaataa aaaactcacc 14460
aaggccatgg caccgagcgt tggttttctt gtattcccct tagtatgcgg cgcgcggcga 14520
tgtatgagga aggtcctcct ccctcctacg agagtgtggt gagcgcggcg ccagtggcgg 14580
cggcgctggg ttctcccttc gatgctcccc tggacccgcc gtttgtgcct ccgcggtacc 14640
tgcggcctac cggggggaga aacagcatcc gttactctga gttggcaccc ctattcgaca 14700
ccacccgtgt gtacctggtg gacaacaagt caacggatgt ggcatccctg aactaccaga 14760
acgaccacag caactttctg accacggtca ttcaaaacaa tgactacagc ccgggggagg 14820

caagcacaca gaccatcaat cttgacgacc ggtcgcactg gggcggcgac ctgaaaacca 14880
tcctgcatac caacatgcca aatgtgaacg agttcatgtt taccaataag tttaaggcgc 14940
gggtgatggt gtcgcgcttg cctactaagg acaatcaggt ggagctgaaa tacgagtggg 15000
tggagttcac gctgcccgag ggcaactact ccgagaccat gaccatagac cttatgaaca 15060
acgcgatcgt ggagcactac ttgaaagtgg gcagacagaa cggggttctg gaaagcgaca 15120
tcggggtaaa gtttgacacc cgcaacttca gactggggtt tgaccccgtc actggtcttg 15180
tcatgcctgg ggtatataca aacgaagcct tccatccaga catcattttg ctgccaggat 15240
gcggggtgga cttcacccac agccgcctga gcaacttgtt gggcatccgc aagcggcaac 15300
ccttccagga gggctttagg atcacctacg atgatctgga gggtggtaac attcccgcac 15360
tgttggatgt ggacgcctac caggcgagct tgaaagatga caccgaacag ggcgggggtg 15420
gcgcaggcgg cagcaacagc agtggcagcg gcgcggaaga gaactccaac gcggcagccg 15480
cggcaatgca gccggtggag gacatgaacg atcatgccat tcgcggcgac acctttgcca 15540
cacgggctga ggagaagcgc gctgaggccg aagcagcggc cgaagctgcc gcccccgctg 15600
cgcaacccga ggtcgagaag cctcagaaga aaccggtgat caaacccctg acagaggaca 15660
gcaagaaacg cagttacaac ctaataagca atgacagcac cttcacccag taccgcagct 15720
ggtaccttgc atacaactac ggcgaccctc agaccggaat ccgctcatgg accctgcttt 15780
gcactcctga cgtaacctgc ggctcggagc aggtctactg gtcgttgcca gacatgatgc 15840
aagaccccgt gaccttccgc tccacgcgcc agatcagcaa ctttccggtg gtgggcgccg 15900
agctgttgcc cgtgcactcc aagagcttct acaacgacca ggccgtctac tcccaactca 15960
tccgccagtt tacctctctg acccacgtgt tcaatcgctt tcccgagaac cagattttgg 16020
cgcgcccgcc agcccccacc atcaccaccg tcagtgaaaa cgttcctgct ctcacagatc 16080
acgggacgct accgctgcgc aacagcatcg gaggagtcca gcgagtgacc attactgacg 16140
ccagacgccg cacctgcccc tacgtttaca aggccctggg catagtctcg ccgcgcgtcc 16200
tatcgagccg cactttttga gcaagcatgt ccatccttat atcgcccagc aataacacag 16260
gctggggcct gcgcttccca agcaagatgt ttggcggggc caagaagcgc tccgaccaac 16320
acccagtgcg cgtgcgcggg cactaccgcg cgccctgggg cgcgcacaaa cgcggccgca 16380
ctgggcgcac caccgtcgat gacgccatcg acgcggtggt ggaggaggcg cgcaactaca 16440
cgcccacgcc gccaccagtg tccacagtgg acgcggccat tcagaccgtg gtgcgcggag 16500
cccggcgcta tgctaaaatg aagagacggc ggaggcgcgt agcacgtcgc caccgccgcc 16560
gacccggcac tgccgcccaa cgcgcggcgg cggccctgct taaccgcgca cgtcgcaccg 16620
gccgacgggc ggccatgcgg gccgctcgaa ggctggccgc gggtattgtc actgtgcccc 16680
ccaggtccag gcgacgagcg gccgccgcag cagccgcggc cattagtgct atgactcagg 16740
gtcgcagggg caacgtgtat tgggtgcgcg actcggttag cggcctgcgc gtgcccgtgc 16800

gcacccgccc cccgcgcaac tagattgcaa gaaaaaacta cttagactcg tactgttgta 16860

tgtatccagc ggcggcggcg cgcaacgaag ctatgtccaa gcgcaaaatc aaagaagaga 16920

tgctccaggt catcgcgccg gagatctatg gccccccgaa gaaggaagag caggattaca 16980

agccccgaaa gctaaagcgg gtcaaaaaga aaaagaaaga tgatgatgat gaacttgacg 17040

acgaggtgga actgctgcac gctaccgcgc ccaggcgacg ggtacagtgg aaaggtcgac 17100

gcgtaaaacg tgttttgcga cccggcacca ccgtagtctt tacgcccggt gagcgctcca 17160

cccgcaccta caagcgcgtg tatgatgagg tgtacggcga cgaggacctg cttgagcagg 17220

ccaacgagcg cctcggggag tttgcctacg gaaagcggca taaggacatg ctggcgttgc 17280

cgctggacga gggcaaccca acacctagcc taaagcccgt aacactgcag caggtgctgc 17340

ccgcgcttgc accgtccgaa gaaaagcgcg gcctaaagcg cgagtctggt gacttggcac 17400

ccaccgtgca gctgatggta cccaagcgcc agcgactgga agatgtcttg gaaaaaatga 17460

ccgtggaacc tgggctggag cccgaggtcc gcgtgcggcc aatcaagcag gtggcgccgg 17520

gactgggcgt gcagaccgtg gacgttcaga tacccactac cagtagcacc agtattgcca 17580

ccgccacaga gggcatggag acacaaacgt ccccggttgc ctcagcggtg gcggatgccg 17640

cggtgcaggc ggtcgctgcg gccgcgtcca agacctctac ggaggtgcaa acggacccgt 17700

ggatgtttcg cgtttcagcc ccccggcgcc cgcgccgttc gaggaagtac ggcgccgcca 17760

gcgcgctact gcccgaatat gccctacatc cttccattgc gcctaccccc ggctatcgtg 17820

gctacaccta ccgccccaga agacgagcaa ctacccgacg ccgaaccacc actggaaccc 17880

gccgccgccg tcgccgtcgc cagcccgtgc tggccccgat ttccgtgcgc agggtggctc 17940

gcgaaggagg caggaccctg gtgctgccaa cagcgcgcta ccaccccagc atcgtttaaa 18000

agccggtctt tgtggttctt gcagatatgg ccctcacctg ccgcctccgt ttcccggtgc 18060

cgggattccg aggaagaatg caccgtagga ggggcatggc cggccacggc ctgacgggcg 18120

gcatgcgtcg tgcgcaccac cggcggcggc gcgcgtcgca ccgtcgcatg cgcggcggta 18180

tcctgcccct ccttattcca ctgatcgccg cggcgattgg cgccgtgccc ggaattgcat 18240

ccgtggcctt gcaggcgcag agacactgat taaaaacaag ttgcatgtgg aaaaatcaaa 18300

ataaaaagtc tggactctca cgctcgcttg gtcctgtaac tattttgtag aatggaagac 18360

atcaactttg cgtctctggc cccgcgacac ggctcgcgcc cgttcatggg aaactggcaa 18420

gatatcggca ccagcaatat gagcggtggc gccttcagct ggggctcgct gtggagcggc 18480

attaaaaatt tcggttccac cgttaagaac tatggcagca aggcctggaa cagcagcaca 18540

ggccagatgc tgagggataa gttgaaagag caaaatttcc aacaaaaggt ggtagatggc 18600

ctggcctctg gcattagcgg ggtggtggac ctggccaacc aggcagtgca aaataagatt 18660

aacagtaagc ttgatccccg ccctcccgta gaggagcctc caccggccgt ggagacagtg 18720

tctccagagg ggcgtggcga aaagcgtccg cgccccgaca gggaagaaac tctggtgacg 18780

caaatagacg agcctccctc gtacgaggag gcactaaagc aaggcctgcc caccacccgt 18840

cccatcgcgc ccatggctac cggagtgctg ggccagcaca cacccgtaac gctggacctg 18900

cctccccccg ccgacaccca gcagaaacct gtgctgccag gcccgaccgc cgttgttgta 18960

acccgtccta gccgcgcgtc cctgcgccgc gccgccagcg gtccgcgatc gttgcggccc 19020

gtagccagtg gcaactggca aagcacactg aacagcatcg tgggtctggg ggtgcaatcc 19080

ctgaagcgcc gacgatgctt ctgatagcta acgtgtcgta tgtgtgtcat gtatgcgtcc 19140

atgtcgccgc cagaggagct gctgagccgc cgcgcgcccg ctttccaaga tggctacccc 19200

ttcgatgatg ccgcagtggt cttacatgca catctcgggc caggacgcct cggagtacct 19260

gagccccggg ctggtgcagt ttgcccgcgc caccgagacg tacttcagcc tgaataacaa 19320

gtttagaaac cccacggtgg cgcctacgca cgacgtgacc acagaccggt cccagcgttt 19380

gacgctgcgg ttcatccctg tggaccgtga ggatactgcg tactcgtaca aggcgcggtt 19440

caccctagct gtgggtgata accgtgtgct ggacatggct tccacgtact ttgacatccg 19500

cggcgtgctg gacaggggcc ctacttttaa gccctactct ggcactgcct acaacgccct 19560

ggctcccaag ggtgccccaa atccttgcga atgggatgaa gctgctactg ctcttgaaat 19620

aaacctagaa gaagaggacg atgacaacga agacgaagta gacgagcaag ctgagcagca 19680

aaaaactcac gtatttgggc aggcgcctta ttctggtata aatattacaa aggagggtat 19740

tcaaataggt gtcgaaggtc aaacacctaa atatgccgat aaaacatttc aacctgaacc 19800

tcaaatagga gaatctcagt ggtacgaaac agaaattaat catgcagctg ggagagtcct 19860

aaaaaagact accccaatga aaccatgtta cggttcatat gcaaaaccca caaatgaaaa 19920

tggagggcaa ggcattcttg taaagcaaca aaatggaaag ctagaaagtc aagtggaaat 19980

gcaatttttc tcaactactg aggcagccgc aggcaatggt gataacttga ctcctaaagt 20040

ggtattgtac agtgaagatg tagatataga aaccccagac actcatattt cttacatgcc 20100

cactattaag gaaggtaact cacgagaact aatgggccaa caatctatgc ccaacaggcc 20160

taattacatt gcttttaggg acaattttat tggtctaatg tattacaaca gcacgggtaa 20220

tatgggtgtt ctggcgggcc aagcatcgca gttgaatgct gttgtagatt tgcaagacag 20280

aaacacagag ctttcatacc agcttttgct tgattccatt ggtgatagaa ccaggtactt 20340

ttctatgtgg aatcaggctg ttgacagcta tgatccagat gttagaatta ttgaaaatca 20400

tggaactgaa gatgaacttc caaattactg ctttccactg ggaggtgtga ttaatacaga 20460

gactcttacc aaggtaaaac ctaaaacagg tcaggaaaat ggatgggaaa aagatgctac 20520

agaattttca gataaaaatg aaataagagt tggaaataat tttgccatgg aaatcaatct 20580

aaatgccaac ctgtggagaa atttcctgta ctccaacata gcgctgtatt tgcccgacaa 20640

gctaaagtac agtccttcca acgtaaaaat ttctgataac ccaaacacct acgactacat 20700

gaacaagcga gtggtggctc ccgggctagt ggactgctac attaaccttg gagcacgctg 20760

gtcccttgac tatatggaca acgtcaaccc atttaaccac caccgcaatg ctggcctgcg 20820

ctaccgctca atgttgctgg gcaatggtcg ctatgtgccc ttccacatcc aggtgcctca 20880

gaagttcttt gccattaaaa acctccttct cctgccgggc tcatacacct acgagtggaa 20940

cttcaggaag gatgttaaca tggttctgca gagctcccta ggaaatgacc taagggttga 21000

cggagccagc attaagtttg atagcatttg cctttacgcc accttcttcc ccatggccca 21060

caacaccgcc tccacgcttg aggccatgct tagaaacgac accaacgacc agtcctttaa 21120

cgactatctc tccgccgcca acatgctcta ccctataccc gccaacgcta ccaacgtgcc 21180

catatccatc ccctcccgca actgggcggc tttccgcggc tgggccttca cgcgccttaa 21240

gactaaggaa accccatcac tgggctcggg ctacgaccct tattacacct actctggctc 21300

tataccctac ctagatggaa ccttttacct caaccacacc tttaagaagg tggccattac 21360

ctttgactct tctgtcagct ggcctggcaa tgaccgcctg cttaccccca acgagtttga 21420

aattaagcgc tcagttgacg gggagggtta caacgttgcc cagtgtaaca tgaccaaaga 21480

ctggttcctg gtacaaatgc tagctaacta taacattggc taccagggct tctatatccc 21540

agagagctac aaggaccgca tgtactcctt ctttagaaac ttccagccca tgagccgtca 21600

ggtggtggat gatactaaat acaaggacta ccaacaggtg ggcatcctac accaacacaa 21660

caactctgga tttgttggct accttgcccc caccatgcgc gaaggacagg cctaccctgc 21720

taacttcccc tatccgctta taggcaagac cgcagttgac agcattaccc agaaaaagtt 21780

tctttgcgat cgcacccttt ggcgcatccc attctccagt aactttatgt ccatgggcgc 21840

actcacagac ctgggccaaa accttctcta cgccaactcc gcccacgcgc tagacatgac 21900

ttttgaggtg gatcccatgg acgagcccac ccttctttat gttttgtttg aagtctttga 21960

cgtggtccgt gtgcaccagc cgcaccgcgg cgtcatcgaa accgtgtacc tgcgcacgcc 22020

cttctcggcc ggcaacgcca caacataaag aagcaagcaa catcaacaac agctgccgcc 22080

atgggctcca gtgagcagga actgaaagcc attgtcaaag atcttggttg tgggccatat 22140

tttttgggca cctatgacaa gcgctttcca ggctttgttt ctccacacaa gctcgcctgc 22200

gccatagtca atacggccgg tcgcgagact gggggcgtac actggatggc ctttgcctgg 22260

aacccgcact caaaaacatg ctacctcttt gagccctttg gcttttctga ccagcgactc 22320

aagcaggttt accagtttga gtacgagtca ctcctgcgcc gtagcgccat tgcttcttcc 22380

cccgaccgct gtataacgct ggaaaagtcc acccaaagcg tacaggggcc caactcggcc 22440

gcctgtggac tattctgctg catgtttctc cacgcctttg ccaactggcc ccaaactccc 22500

atggatcaca accccaccat gaaccttatt accggggtac ccaactccat gctcaacagt 22560

ccccaggtac agcccaccct gcgtcgcaac caggaacagc tctacagctt cctggagcgc 22620

cactcgccct acttccgcag ccacagtgcg cagattagga gcgccacttc tttttgtcac 22680

ttgaaaaaca tgtaaaaata atgtactaga gacactttca ataaaggcaa atgcttttat 22740

ttgtacactc tcgggtgatt atttaccccc acccttgccg tctgcgccgt ttaaaaatca 22800

aaggggttct gccgcgcatc gctatgcgcc actggcaggg acacgttgcg atactggtgt 22860

ttagtgctcc acttaaactc aggcacaacc atccgcggca gctcggtgaa gttttcactc 22920

cacaggctgc gcaccatcac caacgcgttt agcaggtcgg gcgccgatat cttgaagtcg 22980

cagttggggc ctccgccctg cgcgcgcgag ttgcgataca cagggttgca gcactggaac 23040

actatcagcg ccgggtggtg cacgctggcc agcacgctct tgtcggagat cagatccgcg 23100

tccaggtcct ccgcgttgct cagggcgaac ggagtcaact ttggtagctg ccttcccaaa 23160

aagggcgcgt gcccaggctt tgagttgcac tcgcaccgta gtggcatcaa aaggtgaccg 23220

tgcccggtct gggcgttagg atacagcgcc tgcataaaag ccttgatctg cttaaaagcc 23280

acctgagcct ttgcgccttc agagaagaac atgccgcaag acttgccgga aaactgattg 23340

gccggacagg ccgcgtcgtg cacgcagcac cttgcgtcgg tgttggagat ctgcaccaca 23400

tttcggcccc accggttctt cacgatcttg gccttgctag actgctcctt cagcgcgcgc 23460

tgcccgtttt cgctcgtcac atccatttca atcacgtgct ccttatttat cataatgctt 23520

ccgtgtagac acttaagctc gccttcgatc tcagcgcagc ggtgcagcca caacgcgcag 23580

cccgtgggct cgtgatgctt gtaggtcacc tctgcaaacg actgcaggta cgcctgcagg 23640

aatcgcccca tcatcgtcac aaaggtcttg ttgctggtga aggtcagctg caacccgcgg 23700

tgctcctcgt tcagccaggt cttgcatacg gccgccagag cttccacttg gtcaggcagt 23760

agtttgaagt tcgcctttag atcgttatcc acgtggtact tgtccatcag cgcgcgcgca 23820

gcctccatgc ccttctccca cgcagacacg atcggcacac tcagcgggtt catcaccgta 23880

atttcacttt ccgcttcgct gggctcttcc tcttcctctt gcgtccgcat accacgcgcc 23940

actgggtcgt cttcattcag ccgccgcact gtgcgcttac ctcctttgcc atgcttgatt 24000

agcaccggtg ggttgctgaa acccaccatt tgtagcgcca catcttctct ttcttcctcg 24060

ctgtccacga ttacctctgg tgatggcggg cgctcgggct tgggagaagg gcgcttcttt 24120

ttcttcttgg gcgcaatggc caaatccgcc gccgaggtcg atggccgcgg gctgggtgtg 24180

cgcggcacca gcgcgtcttg tgatgagtct tcctcgtcct cggactcgat acgccgcctc 24240

atccgctttt ttgggggcgc ccggggaggc ggcggcgacg gggacgggga cgacacgtcc 24300

tccatggttg ggggacgtcg cgccgcaccg cgtccgcgct cgggggtggt ttcgcgctgc 24360

tcctcttccc gactggccat ttccttctcc tataggcaga aaaagatcat ggagtcagtc 24420

gagaagaagg acagcctaac cgccccctct gagttcgcca ccaccgcctc caccgatgcc 24480

gccaacgcgc ctaccacctt ccccgtcgag gcacccccgc ttgaggagga ggaagtgatt 24540

atcgagcagg acccaggttt tgtaagcgaa gacgacgagg accgctcagt accaacagag 24600

gataaaaagc aagaccagga caacgcagag gcaaacgagg aacaagtcgg gcggggggac 24660

gaaaggcatg gcgactacct agatgtggga gacgacgtgc tgttgaagca tctgcagcgc 24720

cagtgcgcca ttatctgcga cgcgttgcaa gagcgcagcg atgtgcccct cgccatagcg 24780
gatgtcagcc ttgcctacga acgccaccta ttctcaccgc gcgtaccccc caaacgccaa 24840
gaaaacggca catgcgagcc caacccgcgc ctcaacttct accccgtatt tgccgtgcca 24900
gaggtgcttg ccacctatca catctttttc caaaactgca agatacccct atcctgccgt 24960
gccaaccgca gccgagcgga caagcagctg gccttgcggc agggcgctgt catacctgat 25020
atcgcctcgc tcaacgaagt gccaaaaatc tttgagggtc ttggacgcga cgagaagcgc 25080
gcggcaaacg ctctgcaaca ggaaaacagc gaaaatgaaa gtcactctgg agtgttggtg 25140
gaactcgagg gtgacaacgc gcgcctagcc gtactaaaac gcagcatcga ggtcacccac 25200
tttgcctacc cggcacttaa cctacccccc aaggtcatga gcacagtcat gagtgagctg 25260
atcgtgcgcc gtgcgcagcc cctggagagg gatgcaaatt tgcaagaaca aacagaggag 25320
ggcctacccg cagttggcga cgagcagcta gcgcgctggc ttcaaacgcg cgagcctgcc 25380
gacttggagg agcgacgcaa actaatgatg gccgcagtgc tcgttaccgt ggagcttgag 25440
tgcatgcagc ggttctttgc tgacccggag atgcagcgca agctagagga aacattgcac 25500
tacacctttc gacagggcta cgtacgccag gcctgcaaga tctccaacgt ggagctctgc 25560
aacctggtct cctaccttgg aattttgcac gaaaaccgcc ttgggcaaaa cgtgcttcat 25620
tccacgctca agggcgaggc gcgccgcgac tacgtccgcg actgcgttta cttatttcta 25680
tgctacacct ggcagacggc catgggcgtt tggcagcagt gcttggagga gtgcaacctc 25740
aaggagctgc agaaactgct aaagcaaaac ttgaaggacc tatggacggc cttcaacgag 25800
cgctccgtgg ccgcgcacct ggcggacatc attttccccg aacgcctgct taaaaccctg 25860
caacagggtc tgccagactt caccagtcaa agcatgttgc agaactttag gaactttatc 25920
ctagagcgct caggaatctt gcccgccacc tgctgtgcac ttcctagcga ctttgtgccc 25980
attaagtacc gcgaatgccc tccgccgctt tggggccact gctaccttct gcagctagcc 26040
aactaccttg cctaccactc tgacataatg gaagacgtga gcggtgacgg tctactggag 26100
tgtcactgtc gctgcaacct atgcaccccg caccgctccc tggtttgcaa ttcgcagctg 26160
cttaacgaaa gtcaaattat cggtaccttt gagctgcagg gtccctcgcc tgacgaaaag 26220
tccgcggctc cggggttgaa actcactccg gggctgtgga cgtcggctta ccttcgcaaa 26280
tttgtacctg aggactacca cgcccacgag attaggttct acgaagacca atcccgcccg 26340
cctaatgcgg agcttaccgc ctgcgtcatt acccagggcc acattcttgg ccaattgcaa 26400
gccatcaaca aagcccgcca agagtttctg ctacgaaagg gacggggggt ttacttggac 26460
ccccagtccg gcgaggagct caacccaatc cccccgccgc cgcagcccta tcagcagcag 26520
ccgcgggccc ttgcttccca ggatggcacc caaaaagaag ctgcagctgc cgccgccacc 26580
cacggacgag gaggaatact gggacagtca ggcagaggag gttttggacg aggaggagga 26640
ggacatgatg gaagactggg agagcctaga cgaggaagct tccgaggtcg aagaggtgtc 26700

agacgaaaca ccgtcaccct cggtcgcatt cccctcgccg gcgccccaga aatcggcaac 26760
cggttccagc atggctacaa cctccgctcc tcaggcgccg ccggcactgc ccgttcgccg 26820
acccaaccgt agatgggaca ccactggaac cagggccggt aagtccaagc agccgccgcc 26880
gttagcccaa gagcaacaac agcgccaagg ctaccgctca tggcgcgggc acaagaacgc 26940
catagttgct tgcttgcaag actgtggggg caacatctcc ttcgcccgcc gctttcttct 27000
ctaccatcac ggcgtggcct tcccccgtaa catcctgcat tactaccgtc atctctacag 27060
cccatactgc accggcggca gcggcagcaa cagcagcggc cacacagaag caaaggcgac 27120
cggatagcaa gactctgaca aagcccaaga aatccacagc ggcggcagca gcaggaggag 27180
gagcgctgcg tctggcgccc aacgaacccg tatcgacccg cgagcttaga aacaggattt 27240
ttcccactct gtatgctata tttcaacaga gcaggggcca agaacaagag ctgaaaataa 27300
aaaacaggtc tctgcgatcc ctcacccgca gctgcctgta tcacaaaagc gaagatcagc 27360
ttcggcgcac gctggaagac gcggaggctc tcttcagtaa atactgcgcg ctgactctta 27420
aggactagtt tcgcgccctt tctcaaattt aagcgcgaaa actacgtcat ctccagcggc 27480
cacacccggc gccagcacct gttgtcagcg ccattatgag caaggaaatt cccacgccct 27540
acatgtggag ttaccagcca caaatgggac ttgcggctgg agctgcccaa gactactcaa 27600
cccgaataaa ctacatgagc gcgggacccc acatgatatc ccgggtcaac ggaatacgcg 27660
cccaccgaaa ccgaattctc ttggaacagg cggctattac caccacacct cgtaataacc 27720
ttaatccccg tagttggccc gctgccctgg tgtaccagga aagtcccgct cccaccactg 27780
tggtacttcc cagagacgcc caggccgaag ttcagatgac taactcaggg gcgcagcttg 27840
cgggcggctt tcgtcacagg gtgcggtcgc ccgggcaggg tataactcac ctgacaatca 27900
gagggcgagg tattcagctc aacgacgagt cggtgagctc ctcgcttggt ctccgtccgg 27960
acgggacatt tcagatcggc ggcgccggcc gtccttcatt cacgcctcgt caggcaatcc 28020
taactctgca gacctcgtcc tctgagccgc gctctggagg cattggaact ctgcaattta 28080
ttgaggagtt tgtgccatcg gtctacttta accccttctc gggacctccc ggccactatc 28140
cggatcaatt tattcctaac tttgacgcgg taaaggactc ggcggacggc tacgactgaa 28200
tgttaagtgg agaggcagag caactgcgcc tgaaacacct ggtccactgt cgccgccaca 28260
agtgctttgc ccgcgactcc ggtgagtttt gctactttga attgcccgag gatcatatcg 28320
agggcccggc gcacggcgtc cggcttaccg cccagggaga gcttgcccgt agcctgattc 28380
gggagtttac ccagcgcccc ctgctagttg agcgggacag gggaccctgt gttctcactg 28440
tgatttgcaa ctgtcctaac cttggattac atcaagatcc tctagttaat gtcgacttcg 28500
cgatggatcc attaactaat aaaaaaaaat aataaagcat cacttactta aaatcagtta 28560
gcaaatttct gtccagttta ttcagcagca cctccttgcc ctcctcccag ctctggtatt 28620
gcagcttcct cctggctgca aactttctcc acaatctaaa tggaatgtca gtttcctcct 28680

gttcctgtcc atccgcaccc actatcttca tgttgttgca gatgaagcgc gcaagaccgt 28740

ctgaagatac cttcaacccc gtgtatccat atgacacgga aaccggtcct ccaactgtgc 28800

cttttcttac tcctcccttt gtatccccca atgggtttca agagagtccc cctggagttc 28860

ttactttaaa atgtttaacc ccactaacaa ccacaggcgg atctctacag ctaaaagtgg 28920

gagggggact tacagtggat gacactgatg gtaccttaca agaaaacata cgtgctacag 28980

cacccattac taaaaataat cactctgtag aactatccat tggaaatgga ttagaaactc 29040

aaaacaataa actatgtgcc aaattgggaa atgggttaaa atttaacaac ggtgacattt 29100

gtataaagga tagtattaac accttatgga ctggaataaa ccctccacct aactgtcaaa 29160

ttgtggaaaa cactaataca aatgatggca aacttacttt agtattagta aaaaacggag 29220

ggcttgttaa tggctacgtg tctctagttg gtgtatcaga cactgtgaac caaatgttca 29280

cacaaaagac agcaaacatc caattaagat tatattttga ctcttctgga aatctattaa 29340

ctgatgaatc agacttaaaa attccactta aaaataaatc ttctacagcg accagtgaaa 29400

ctgtagccag cagcaaagcc tttatgccaa gtactacagc ttatcccttc aacaccacta 29460

ctagggatag tgaaaactac attcatggaa tatgttacta catgactagt tatgatagaa 29520

gtctatttcc cttgaacatt tctataatgc taaacagccg tatgatttct tccaatgttg 29580

cctatgccat acaatttgaa tggaatctaa atgcaagtga atctccagaa agcaacatag 29640

ctacgctgac cacatccccc tttttctttt cttacattac agaagacgac aactaaaatg 29700

cccaagaata aagaatcgtt tgtgttatgt ttcaacgtgt ttatttttca attgcagaaa 29760

atttcaagtc atttttcatt cagtagtata gccccaccac cacatagctt atacagatca 29820

ccgtacctta atcaaactca cagaacccta gtattcaacc tgccacctcc ctcccaacac 29880

acagagtaca cagtcctttc tccccggctg gccttaaaaa gcatcatatc atgggtaaca 29940

gacatattct taggtgttat attccacacg gtttcctgtc gagccaaacg ctcatcagtg 30000

atattaataa actccccggg cagctcactt aagttcatgt cgctgtccag ctgctgagcc 30060

acaggctgct gtccaacttg cggttgctta acgggcggcg aaggagaagt ccacgcctac 30120

atgggggtag agtcataatc gtgcatcagg atagggcggt ggtgctgcag cagcgcgcga 30180

ataaactgct gccgccgccg ctccgtcctg caggaataca acatggcagt ggtctcctca 30240

gcgatgattc gcaccgcccg cagcataagg cgccttgtcc tccgggcaca gcagcgcacc 30300

ctgatctcac ttaaatcagc acagtaactg cagcacagca ccacaatatt gttcaaaatc 30360

ccacagtgca aggcgctgta tccaaagctc atggcgggga ccacagaacc cacgtggcca 30420

tcataccaca agcgcaggta gattaagtgg cgacccctca taaacacgct ggacataaac 30480

attacctctt ttggcatgtt gtaattcacc acctcccggt accatataaa cctctgatta 30540

aacatggcgc catccaccac catcctaaac cagctggcca aaacctgccc gccggctata 30600

cactgcaggg aaccgggact ggaacaatga cagtggagag cccaggactc gtaaccatgg 30660

atcatcatgc tcgtcatgat atcaatgttg gcacaacaca ggcacacgtg catacacttc 30720

ctcaggatta caagctcctc ccgcgttaga accatatccc agggaacaac ccattcctga 30780

atcagcgtaa atcccacact gcagggaaga cctcgcacgt aactcacgtt gtgcattgtc 30840

aaagtgttac attcgggcag cagcggatga tcctccagta tggtagcgcg ggtttctgtc 30900

tcaaaaggag gtagacgatc cctactgtac ggagtgcgcc gagacaaccg agatcgtgtt 30960

ggtcgtagtg tcatgccaaa tggaacgccg gacgtagtca tatttcctga agcaaaacca 31020

ggtgcgggcg tgacaaacag atctgcgtct ccggtctcgc cgcttagatc gctctgtgta 31080

gtagttgtag tatatccact ctctcaaagc atccaggcgc cccctggctt cgggttctat 31140

gtaaactcct tcatgcgccg ctgccctgat aacatccacc accgcagaat aagccacacc 31200

cagccaacct acacattcgt tctgcgagtc acacacggga ggagcgggaa gagctggaag 31260

aaccatgttt ttttttttat tccaaaagat tatccaaaac ctcaaaatga agatctatta 31320

agtgaacgcg ctcccctccg gtggcgtggt caaactctac agccaaagaa cagataatgg 31380

catttgtaag atgttgcaca atggcttcca aaaggcaaac ggccctcacg tccaagtgga 31440

cgtaaaggct aaacccttca gggtgaatct cctctataaa cattccagca ccttcaacca 31500

tgcccaaata attctcatct cgccaccttc tcaatatatc tctaagcaaa tcccgaatat 31560

taagtccggc cattgtaaaa atctgctcca gagcgccctc caccttcagc ctcaagcagc 31620

gaatcatgat tgcaaaaatt caggttcctc acagacctgt ataagattca aaagcggaac 31680

attaacaaaa ataccgcgat cccgtaggtc ccttcgcagg gccagctgaa cataatcgtg 31740

caggtctgca cggaccagcg cggccacttc cccgccagga accttgacaa aagaacccac 31800

actgattatg acacgcatac tcggagctat gctaaccagc gtagccccga tgtaagcttt 31860

gttgcatggg cggcgatata aaatgcaagg tgctgctcaa aaaatcaggc aaagcctcgc 31920

gcaaaaaaga aagcacatcg tagtcatgct catgcagata aaggcaggta agctccggaa 31980

ccaccacaga aaaagacacc atttttctct caaacatgtc tgcgggtttc tgcataaaca 32040

caaaataaaa taacaaaaaa acatttaaac attagaagcc tgtcttacaa caggaaaaac 32100

aacccttata agcataagac ggactacggc catgccggcg tgaccgtaaa aaaactggtc 32160

accgtgatta aaaagcacca ccgacagctc ctcggtcatg tccggagtca taatgtaaga 32220

ctcggtaaac acatcaggtt gattcatcgg tcagtgctaa aaagcgaccg aaatagcccg 32280

ggggaataca tacccgcagg cgtagagaca acattacagc ccccatagga ggtataacaa 32340

aattaatagg agagaaaaac acataaacac ctgaaaaacc ctcctgccta ggcaaaatag 32400

caccctcccg ctccagaaca acatacagcg cttcacagcg gcagcctaac agtcagcctt 32460

accagtaaaa aagaaaacct attaaaaaaa caccactcga cacggcacca gctcaatcag 32520

tcacagtgta aaaaagggcc aagtgcagag cgagtatata taggactaaa aaatgacgta 32580

acggttaaag tccacaaaaa acacccagaa aaccgcacgc gaacctacgc ccagaaacga 32640

aagccaaaaa acccacaact tcctcaaatc gtcacttccg ttttcccacg ttacgtaact 32700

tcccatttta agaaaactac aattcccaac acatacaagt tactccgccc taaaacctac 32760

gtcacccgcc ccgttcccac gccccgcgcc acgtcacaaa ctccaccccc tcattatcat 32820

attggcttca atccaaaata aggtatatta ttgatgatgt taattaattt cgaacccggg 32880

gtaccgaatt cctcgagtct agaggagcat gcgacgtcgc aattcgccct atagtgagtc 32940

gtattacaat tcactggccg tcgttttaca acgtcgtgac tgggaaaacc ctggcgttac 33000

ccaacttaat cgccttgcag cacatccccc tttcgccagc tggcgtaata gcgaagaggc 33060

ccgcaccgat cgcccttccc aacagttgcg cagcctgaat ggcgaatgga aattgtaagc 33120

gttaatattt tgttaaaatt cgcgttaaat ttttgttaaa tcagctcatt ttttaaccaa 33180

taggccgaaa tcggcaaaat cccttataaa tcaaaagaat agaccgagat agggttgagt 33240

gttgttccag tttggaacaa gagtccacta ttaaagaacg tggactccaa cgtcaaaggg 33300

cgaaaaaccg tctatcaggg cgatggccca ctacgtgaac catcacccta atcaagtttt 33360

ttggggtcga ggtgccgtaa agcactaaat cggaacccta aagggagccc ccgatttaga 33420

gcttgacggg gaaagccggc gaacgtggcg agaaaggaag ggaagaaagc gaaaggagcg 33480

ggcgctaggg cgctggcaag tgtagcggtc acgctgcgcg taaccaccac acccgccgcg 33540

cttaatgcgc cgctacaggg cgcgtcctga tgcggtattt tctccttacg catctgtgcg 33600

gtatttcaca ccgcatacag gtggcacttt tcggggaaat gtgcgcggaa cccctatttg 33660

tttatttttc taaatacatt caaatatgta tccgctcatg agacaataac cctgataaat 33720

gcttcaataa tattgaaaaa ggaagagtat gagtattcaa catttccgtg tcgcccttat 33780

tccctttttt gcggcatttt gccttcctgt ttttgctcac ccagaaacgc tggtgaaagt 33840

aaaagatgct gaagatcagt tgggtgcacg agtgggttac atcgaactgg atctcaacag 33900

cggtaagatc cttgagagtt ttcgccccga agaacgtttt ccaatgatga gcacttttaa 33960

agttctgcta tgtggcgcgg tattatcccg tattgacgcc gggcaagagc aactcggtcg 34020

ccgcatacac tattctcaga atgacttggt tgagtactca ccagtcacag aaaagcatct 34080

tacggatggc atgacagtaa gagaattatg cagtgctgcc ataaccatga gtgataacac 34140

tgcggccaac ttacttctga caacgatcgg aggaccgaag gagctaaccg cttttttgca 34200

caacatgggg gatcatgtaa ctcgccttga tcgttgggaa ccggagctga atgaagccat 34260

accaaacgac gagcgtgaca ccacgatgcc tgtagcaatg gcaacaacgt tgcgcaaact 34320

attaactggc gaactactta ctctagcttc ccggcaacaa ttaatagact ggatggaggc 34380

ggataaagtt gcaggaccac ttctgcgctc ggcccttccg gctggctggt ttattgctga 34440

taaatctgga gccggtgagc gtgggtctcg cggtatcatt gcagcactgg ggccagatgg 34500

taagccctcc cgtatcgtag ttatctacac gacggggagt caggcaacta tggatgaacg 34560

aaatagacag atcgctgaga taggtgcctc actgattaag cattggtaac tgtcagacca 34620

agtttactca tatatacttt agattgattt aaaacttcat ttttaattta aaaggatcta 34680

ggtgaagatc ctttttgata atctcatgac caaaatccct taacgtgagt tttcgttcca 34740

ctgagcgtca gaccccgtag aaaagatcaa aggatcttct tgagatcctt tttttctgcg 34800

cgtaatctgc tgcttgcaaa caaaaaaacc accgctacca gcggtggttt gtttgccgga 34860

tcaagagcta ccaactcttt ttccgaaggt aactggcttc agcagagcgc agataccaaa 34920

tactgttctt ctagtgtagc cgtagttagg ccaccacttc aagaactctg tagcaccgcc 34980

tacatacctc gctctgctaa tcctgttacc agtggctgct gccagtggcg ataagtcgtg 35040

tcttaccggg ttggactcaa gacgatagtt accggataag gcgcagcggt cgggctgaac 35100

ggggggttcg tgcacacagc ccagcttgga gcgaacgacc tacaccgaac tgagatacct 35160

acagcgtgag ctatgagaaa gcgccacgct tcccgaaggg agaaaggcgg acaggtatcc 35220

ggtaagcggc agggtcggaa caggagagcg cacgagggag cttccagggg gaaacgcctg 35280

gtatctttat agtcctgtcg ggtttcgcca cctctgactt gagcgtcgat ttttgtgatg 35340

ctcgtcaggg gggcggagcc tatggaaaaa cgccagcaac gcggcctttt tacggttcct 35400

ggccttttgc tggccttttg ctcacatgtt ctttcctgcg ttatcccctg attctgtgga 35460

taaccgtatt accgcctttg agtgagctga taccgctcgc cgcagccgaa cgaccgagcg 35520

cagcgagtca gtgagcgagg aagcggaaga gcgcccaata cgcaaaccgc ctctccccgc 35580

gcgttggccg attcattaat gcagctggca cgacaggttt cccgactgga aagcgggcag 35640

tgagcgcaac gcaattaatg tgagttagct cactcattag gcaccccagg ctttacactt 35700

tatgcttccg gctcgtatgt tgtgtggaat tgtgagcgga taacaatttc acacaggaaa 35760

cagctatgac catgattacg ccaagctatt taggtgacac tatagaatac tcaagctagt 35820

taattaacgt taattaacat catcaataat ataccttatt ttggattgaa gccaatatga 35880

taatgagggg gtggagtttg tgacgtggcg cggggcgtgg gaacggggcg ggtgacgtag 35940

tagtgtggcg gaagtgtgat gttgcaagtg tggcggaaca catgtaagcg acggatgtgg 36000

caaaagtgac gtttttggtg tgcgccggtg tacacaggaa gtgacaattt tcgcgcggtt 36060

ttaggcggat gttgtagtaa atttgggcgt aaccgagtaa gatttggcca ttttcgcggg 36120

aaaactgaat aagaggaagt gaaatctgaa taattttgtg ttactcatag cgcgtaatct 36180

ctagcat 36187

## Description

### Technical Field

The present invention relates to chimeric adenoviruses, into which any transcriptional regulatory domain is introduced, having gene transfer efficiency enhanced by replacing only an adenovirus type 5 fiber knob domain by a corresponding domain of adenovirus type 35 and capable of controlling expression of adenovirus E1A and E1B genes, to methods of producing the chimeric adenoviruses, and to medicaments using them.

### Background Art

Adenoviruses are frequently used in, e.g., *in vitro* and *in vivo* analyses of gene functions, due to having high gene expression efficiency compared to other virus vectors or nonvirus vectors. Currently, 51 types of adenoviruses have been known, among which adenovirus type 5 is frequently used as a vector for gene transfer due to, in addition to all the revealed base sequences of the type 5, the small genome size of the virus, which relatively facilitates genetic recombination in use as a vector. In addition, the type 5 has been found to exhibit no carcinogenic properties in human on a number of epidemiological studies. Known diseases caused by the virus type 5 in human include upper respiratory tract inflammation, and the virus is known to be a so-called cold virus. In addition, the biological characteristics of the virus have been adequately analyzed to reveal that the virus can be relatively safely used for human. In addition, for example, non-propagative viruses type 5 defective in E1A and E1B genes as the early-response genes of the viruses type 5 are widely applied as gene therapy vectors used in administration of genes to human bodies.

The infectivity of adenovirus to target cells depends primarily on binding of its fiber knob domain to a receptor on the cells. In case of the adenovirus type 5, coxsakievirus-adenovirus receptor (CAR) is a receptor on the cells (Non-Patent Document 1). In addition, binding of the penton base domain of the virus to integrin molecules on cells is also involved in the infectivity of the virus to the cells; however, since a degree of its contribution to the infection is low compared to the binding of the fiber knob domain to CAR molecules, the infectivity of the type 5 greatly depends on expression of the CAR molecules on the target cells. However, the expression of CAR molecules is often decreased in tumor cells or the like (Non-Patent Document 2), and the CAR expression is also significantly decreased in myeloid cells or the like even if the cells are normal (Non-Patent Document 1), resulting in the decreased efficiency of gene transfer into these target cells by the virus type 5.

Techniques for solving such problems, which have been used, include two techniques that artificially modify a fiber knob domain: a first technique uses a sequence binding specific molecules, such as an Arg-Gly-Asp (RGD) sequence, (Non-Patent Document 2); and a second technique replaces the domain by the fiber knob domain of another virus type. The first technique, which utilizes the ability of the RGD sequence to bind integrins alpha v beta 3 or alpha v beta 5, secondary receptors for type 5, has the following remaining several problems: the RGD sequence inserted into the fiber domain is not a native virus structural protein; gene transfer efficiency also depends on the integrin expression of target cells; and the like. The second technique utilizes a fiber knob domain of adenovirus type 3 belonging to subtype B1 (Non-Patent Document 3) or a fiber knob domain of adenovirus type 11 or 35 belonging to subtype B2 (Non-Patent Document 1). Type 3 receptors have been recently found to be CD80 and CD86 (Non-Patent Document 4). Although such molecules are expressed generally on immunocompetent cells and have a different range of target cells from that of the type 5, they allow no notable improvement in gene transfer efficiency in a wide range of cells.

A cell receptor for adenovirus type 11 or 35, however, has been found to be CD46 (Non-Patent Document 5), more specifically, exhibiting that, since a type 11 or 35 fiber knob domain binds CD46 molecules, the infectivity of the virus depends primarily on the expression of CD46 on target cells. CD46 is known to have a function of inhibiting activation of a complement and to be widely expressed in human cells excluding red blood cells, highly expressed in tumor cells. A known disease caused by the type 11 or 35 in human is urinary tract infection; however, no detailed analysis of the pathogenicity of the adenovirus nor clarification of its biochemical characteristics in terms of a virus has accomplished. Accordingly, although use of the virus type 11 or 35 without being processed has remaining problems, a chimeric vector with only a type 5 fiber knob domain converted into a type 11 or 35 allows gene transfer into a wide range of cells while capitalizing on the characteristic of the type 5 having no carcinogenic properties in human and is anticipated to be a tool for efficient gene transfer when being targeted to cells with deceased CAR expression in particular.

Therefore, actual examination of the efficiency of gene transfer into various tumor cells and normal cells using virus (Avior Therapeutics Inc., Seattle, U.S.A.), which controls expression of Green Fluorescence Protein (GFP) by a cytomegalovirus promoter, by the non-propagative viruses type 5 with the replaced fiber knob domains, revealed that the type 11 has higher gene transfer efficiency than that of the type 5 and the type 35 has higher gene transfer efficiency than that of the type 11 (Non-Patent Document 6). In addition, cells such as peripheral blood, dendritic and CD34-positive bone marrow stem cells are not infected with the virus type 5 at all, while adenovirus using a type 35 fiber knob domain allows gene transfer (Non-Patent Document 1). Accordingly, the adenovirus type 5, which is a currently mainstream vector in gene transfer, is considered to have usefulness limited to a certain degree judging from the efficiency of infection into target cells, whereas the chimeric virus that utilizes the type 35 fiber knob domain allowing the efficiency of gene transfer into a wide range is considered to be useful.

A wild-type adenovirus, however, has high cytotoxic activity, which depends on expression of E1A and E1B which are the early-response genes of the virus. This is because the E1A and E1B genes are deeply involved in the cell cycle of infected cells, regulation of protein synthesis, and virus growth. Accordingly, a virus defective in the genes, which do not grow in infected cells to make it non-propagative, is used as a vector for gene transfer. However, the expression of the E1A and E1B genes in the infected cells results in initiation of virus growth to finally kill the infected cells. In other words, this exhibits that the specificity of cells to be damaged can be regulated by controlling the expression of the E1A and E1B genes in a particular cell population. For example, by controlling the expression of the E1A and E1B genes using the transcriptional regulatory domains of genes which are highly expressed in tumor cells, this recombinant virus is expected to be an oncolytic virus which is lysed specifically in tumor. Such known transcriptional regulatory domains comparatively specific to tumor cells include promoters of midkine (Non-Patent Document 7), survivin (Non-Patent Document 8), cyclooxygenase-2 (Non-Patent Document 9) and the like. Production of adenoviruses having transcriptional regulatory domains, into which such tumor promoters were inserted, resulted in confirmation that the viruses powerfully killed tumor cells even *in vivo* (Non-Patent Document 10). In other words, these recombinant viruses have been found to have cell cytotoxicity determined by the specificity of the transcriptional regulatory domains and to damage human tumor to exhibit anti-tumor activity (Non-Patent Document 11).

However, these conventional cytolytic viruses had such a disadvantage that, because all the viruses used viruses type 5, the infectivity of the viruses was not always high when targeting cells with low expression of CAR, resulting in the impossibility of obtaining sufficient anti-tumor activity. It was conceivable that, because expression of CAR was often low in actual human tumors, use of a high-titer virus solution having multiplicity of infection (MOI) was required for obtaining anti-tumor activity by the viruses type 5, resulting in onset of side effects such as cytotoxic effect due to administration of a large dose of the viruses. Accordingly, enhanced infectivity to target cells must allow more effective damage to the cells of interest. Therefore, a cytolytic virus was produced by modifying type 5 using a type 3 fiber knob domain, and it was found to have usefulness (Non-Patent Document 12).

In addition, a conventional method of producing adenoviruses had such a disadvantage that it was inefficient because of the method comprising introducing plasmid DNA containing a plurality of adenovirus genes into *E*. *coli* or HEK293 cells as virus-producing cells and depending on a homologous gene recombination mechanism in the cells, and the step for isolating a recombinant required a number of procedures, resulting in frequent difficulty in production of a chimeric virus. However, a method by Mizuguchi et al., comprising linking two DNAs of a virus vector in *E. coli* and transfecting a unit of the DNAs of all adenoviruses into HEK293 cells, has been considered to be easy compared to the conventional method (Non-Patent Document 13).

Non-Patent Document 1: Shayakhmetov DM et al., J Virol, 74: 2567-2583, 2000.
Non-Patent Document 2: Dehari H et al., Cancer Gene Ther, 10: 75-85, 2003.
Non-Patent Document 3: Kanerva A et al., Clin Cancer Res, 8: 275-280, 2002.
Non-Patent Document 4: Short JJ et al., Virology, 322: 349-359, 2004.
Non-Patent Document 5: Gaggar A et al., Nat Med, 9: 1408-1412, 2003.
Non-Patent Document 6: Yu L et al., Oncol Rep, 14: 831-835, 2005.
Non-Patent Document 7: Miyauchi M et al., Int J Cancer, 91: 723-727, 2001.
Non-Patent Document 8: Bao R et al., J Natl Cancer Inst, 94: 522-528, 2002.
Non-Patent Document 9: Yamamoto M et al., Mol Ther, 3: 385-394, 2001.
Non-Patent Document 10: DeWeese TL et al., Cancer Res, 61: 7464-7472, 2001.
Non-Patent Document 11: Yoon TL et al., Curr Cancer Drug Targets, 1: 85-107, 2001.
Non-Patent Document 12: Bauerschmitz GJ et al., Mol Ther, 14: 164-174, 2006.
Non-Patent Document 13: Mizuguchi H et al., Hum Gene Ther, 9: 2577-2583, 1998.

### Disclosure of Invention

### Problems to be Solved by the Invention

Because a conventional adenovirus vector is type 5, its infection efficiency depends on expression of a CAR molecule which is a virus receptor, and, consequently, efficiency of gene transfer into a target cell will depend on CAR expression on the cell. Accordingly, in cells with low CAR expression, the efficiency of infection with the virus type 5 is known to decrease to result in low gene transfer efficiency, and the virus type 5 does not act as a vector when targeting, e.g., bone marrow cells for gene transfer. Therefore, replacement of a fiber knob domain that is based on the type 5 having definite biology functions and no carcinogenicity and is a receptor binding domain by that of an adenovirus having a molecule, expressed in a wide range of cells, as a receptor, allows targeting of more cells without being limited by CAR expression, and infection at low MOI in comparison with the virus type 5, resulting in possible avoidance of side effects due to the adenovirus. A similar chimeric virus using a type 3 fiber knob domain has been reported; however, since the receptor expression of the type 3 is comparatively limited as described above, the virus tends to be unsuitable for targeting a wide range of cells. Therefore, a chimeric virus having a domain replaced by a corresponding domain of a type 35 with CD46, which is expressed on human cells excluding red blood cells and is highly expressed in human tumor, as a receptor, must be excellent in gene transfer into target cells compared to the virus type 5. Therefore, the present inventors focused attention on the production of the cytolytic viruses having the type 35 fiber knob domain using the virus type 5 (chimeric virus produced by modifying the type 5).

However, the production of such chimeric viruses was conventionally carried out by a method based on a homologous gene recombination mechanism in *E. coli* or HEK293 cells because of no suitable vector DNA therefor, and therefore was often difficult. Also, it was difficult to produce a chimeric virus by a method by Mizuguchi et al. (Non-Patent Document 13) because of no suitable vector DNA.

It is accordingly an object of the present invention to provide a method of efficiently producing a chimeric adenovirus by modifying a type 5 using vector DNA in which only an adenovirus type 5 fiber knob domain is replaced by the corresponding domain of an adenovirus type 35 to enhance gene transfer efficiency, and using vector DNA produced by introducing any transcriptional regulatory domain, which can control expression of adenovirus E1A and E1B genes, into a region from which a type 5 E1A transcriptional regulatory domain has been removed. It is still another object of the present invention to provide such a chimeric adenovirus or to provide a medicament to which it is applied.

### Means for Solving Problem

An embodiment in accordance with claim 1 of the present invention is a chimeric adenovirus, in which a fiber knob domain in an adenovirus type 5 is replaced by an adenovirus type 35 fiber knob domain and any extraneous transcriptional regulatory domain controlling expression of genes E1A and E1B is introduced into a region from which a type 5 E1A transcriptional regulatory domain has been removed.

An embodiment in accordance with claim 2 is the chimeric adenovirus according to claim 1, wherein the extraneous transcriptional regulatory domain is a tissue-specific promoter. In addition, the chimeric adenovirus provided herein is a cytolytic virus that lyses a target cell.

An embodiment in accordance with claim 3 is the chimeric adenovirus according to claim 1, wherein the extraneous transcriptional regulatory domain is a tumor promoter. In addition, the chimeric adenovirus provided herein is an oncolytic virus that lyses a target tumor cell.

An embodiment in accordance with claim 4 is the chimeric adenovirus according to claim 3, wherein the tumor promoter is a promoter of a midkine, survivin or cyclooxygenase-2 gene.

An embodiment in accordance with claim 5 is the chimeric adenovirus according to claim 1, comprising a base sequence provided by introducing a base sequence encoding any extraneous transcriptional regulatory domain into a base sequence as shown in SEQ ID NO. 3 in the sequence listing or a base sequence homologous thereto. As used herein, a homologous base sequence means a base sequence having characteristics substantially similar to those of a general base sequence, wherein one or several tens of bases are deleted, replaced, inserted or added.

An embodiment in accordance with claim 6 is a method of producing a chimeric adenovirus, comprising using one vector DNA made by linking a fragment including a base sequence encoding an adenovirus obtained by vector DNA (1), in which an adenovirus type 5 fiber knob domain is replaced by an adenovirus type 35 fiber knob domain, to a fragment including: any extraneous transcriptional regulatory domain provided by produced vector DNA (2'), in which any extraneous transcriptional regulatory domain is introduced into vector DNA (2) for use in introduction of any extraneous transcriptional regulatory domain that controls expression of adenovirus type 5 genes E1A and E1B into a region from which an adenovirus type 5 E1A transcriptional regulatory domain has been removed; and a base sequence encoding E1A and E1B, when producing the chimeric adenovirus according to claim 1.

An embodiment in accordance with claim 7 is the method of producing the chimeric adenovirus according to claim 6, wherein the vector DNA (1) is vector DNA (pAd5F35) having a base sequence as shown in SEQ ID NO. 1 in the sequence listing or a base sequence homologous thereto.

An embodiment in accordance with claim 8 is the method of producing the chimeric adenovirus according to claim 6 or 7, wherein the vector DNA (2) is vector DNA (pS-PL/E1A-E1B) having a base sequence as shown in SEQ ID NO. 2 in the sequence listing or a base sequence homologous thereto.

An embodiment in accordance with claim 9 is a medicament comprising: a chimeric adenovirus, in which a fiber knob domain in an adenovirus type 5 is replaced by an adenovirus type 35 fiber knob domain and any extraneous transcriptional regulatory domain controlling expression of E1A and E1B genes is introduced into a region from which a type 5 E1A transcriptional regulatory domain has been removed; or a cell infected with the virus.

In addition, an embodiment in accordance with claim 10 is the medicament according to claim 9, wherein the chimeric adenovirus is a cytolytic virus that lyses a target cell or an oncolytic virus that lyses a target tumor cell.

### Effect of the Invention

A chimeric adenovirus, which has a structure based on an adenovirus type 5 having confirmed safety and revealed biochemical characteristics and in which only a fiber knob domain is replaced by a type 35, facilitates gene transfer into cells with low CAR expression, such as human tumor cells and hematocytic cells, compared to the type 5. In accordance with the present invention, type 5-modified vector DNA having the type 35 fiber knob domain and vector DNA which can easily control expression of E1A and E1B genes were produced using an extraneous transcriptional regulatory domain, and the chimeric adenovirus was able to be produced using these vector systems. In addition, gene transfer by such a chimeric adenovirus was enabled in a number of cells, and, in particular, the gene transfer into cells, which had not been able to be carried out by a conventional adenovirus type 5, was easily enabled. Use of these vector systems markedly facilitates production of chimeric cytolytic viruses compared to conventional methods, and the chimeric cytolytic viruses produced by the vector systems have efficacy against a wide range of tumors and a high anti-tumor activity compared to a conventional type 5 oncolytic virus or the like and thus are useful as medicaments against a number of intractable tumors.

### Brief Description of the Drawings

FIG. 1 is a view showing mean fluorescence intensities at 2 days after washing human malignant mesothelioma and human liver carcinoma cells infected with Ad5-GFP and Ad5F35-GFP at a MOI of 3 or 30.
FIG. 2 is a view showing mean fluorescence intensities at 2 days after washing human esophageal carcinoma, human liver carcinoma and HEK293 cells infected with Ad5-GFP and Ad5F35-GFP at a MOI of 3 or 30.
FIG. 3 is a view showing the results of growth of Ad5/MK, Ad5F35/MK, Ad5/Sur and Ad5F35/Sur in human malignant mesothelioma MSTO-211H cells.
FIG. 4 is a view showing the anti-tumor activities of chimeric cytolysis adenoviruses against human malignant mesothelioma MSTO-211H cells inoculated into nude mice.

### Best Mode for Carrying Out the Invention

In accordance with the present invention, for example, vector DNA (pAd5F35), in which a region corresponding to an adenovirus type 5 fiber knob domain is replaced by a corresponding type 35 domain, is produced. Meanwhile, a multicloning site and vector DNA (pS-PL/E1A-E1B) having E1A and E1B genes in the 3' downstream thereof are produced in a region, from which a type 5 E1A transcriptional regulatory domain has been removed, any transcriptional regulatory domain is inserted into the multicloning site of the pS-PL/E1A-E1B, the DNA fragments of the pS-PL/E1A-E1B excised using appropriate restriction enzyme sites are inserted into the pAd5F35 to make one DNA, and thereafter gene transfer into HEK293 or other cells is carried out. As a result, a type 5-modified chimeric adenovirus, in which E1A and E1B genes are controlled in any extraneous transcriptional regulatory domain and a fiber knob domain is replaced with a 35 type, was found to be surely produced from cells with observed cytopathic effects (CPE).

In this method, there is no necessity for cloning plaques with CPE each time to examine whether they are viruses to be sought, in comparison with conventional methods of producing an adenovirus, and all obtained plaques are viruses. Accordingly, a chimeric adenovirus, which has cytotoxic activity depending on the specificity of any extraneous transcriptional regulatory domain and a type 35 fiber knob domain can be surely and markedly easily produced.

As any extraneous transcriptional regulatory domain, a tissue-specific promoter or a tumor promoter may be used. For the tumor promoter, a promoter of a midkine, survivin or cyclooxygenase-2 gene is preferred. When a tumor promoter is used as any extraneous transcriptional regulatory domain, an obtained chimeric adenovirus is specific to a tumor and shows cytotoxic activity and, in particular, has efficacy against tumors with low CAR expression.

Vector systems necessary for production of a chimeric adenovirus according to the present invention, for example, are pAd5F35 (32,407 bp) with a base sequence as shown in SEQ ID NO. 1 and pS-PL/E1A-E1B (6,663 bp) as shown in SEQ ID NO. 2. The base sequence of-pAd5F35 is in an adenovirus type 5 (NCBI Accession Number: M73260) defective generally in E1 and E3 domains, in which a base sequence portion of a type 5 CAR binding domain is replaced by a base sequence portion exhibiting a type 35 CD46 binding domain. The pS-PL/E1A-E1B, which is also used for production of a conditional replication-competent adenovirus, is inserted into an extraneous transcriptional regulatory domain to express E1A and E1B genes depending on the characteristics of their transcriptional regulatory domains and is used with pAd5F35 in production of cytolytic viruses. A base sequence as shown in SEQ ID NO. 3 (36,187 bp) is one, which is obtained from the base sequences in SEQ ID NOs. 1 and 2 and into which any extraneous transcriptional regulatory domain has not been inserted. In accordance with the present invention, particularly preferred is a chimeric adenovirus having a base sequence obtained by introducing a base sequence encoding any extraneous transcriptional regulatory domain into the base sequence as shown in SEQ ID NO. 3 in the sequence listing or a base sequence homologous thereto.

The present invention is not limited to the above-mentioned SEQ ID NO. 1 but includes one, in which any region in a fragment 31042-32787 corresponding to a fiber knob domain in Accession Number: M73260 is replaced by any region of a fragment 30827-33609 corresponding to an adenovirus type 35 fiber knob domain as shown in NCBI Accession Number: AY271307. In addition, the present invention also includes a vector system, which is hybridized with the base sequences as shown in SEQ ID NOs. 1 and 2 on stringent conditions, as in case of the base sequences as shown in SEQ ID NOs. 1 and 2, can encode a chimeric adenovirus type 35 fiber knob protein and an adenovirus type 5 protein of the other region in SEQ ID NO. 1, controls expression of E1A and E1B genes in any transcriptional regulatory domain in SEQ ID NO. 2, and finally makes these DNAs to one DNA strand in *E. coli.*

Those skilled in the art can easily perform the above-mentioned genetic recombination following, e.g., fundamental books such as Molecular Cloning 2nd Edt., Cold Spring Harbor Laboratory Press(1989), and typical molecular biological techniques such as a PCR method and a method of gene transfer into cultured cells referring to fundamental books such as Molecular Cloning as described above.

A chimeric adenovirus according to the present invention can be easily produced by a well-known gene recombination technique using one DNA, which is made by: first producing vector DNA (1), in which an adenovirus type 5 fiber knob domain is replaced by an adenovirus type 35 fiber knob domain, and vector DNA (2'), in which any extraneous transcriptional regulatory domain is introduced into vector DNA (2) for use in introduction of any extraneous transcriptional regulatory domain, which controls expression of adenovirus type 5 E1A and E1B genes, into a region from which an adenovirus type 5 E1A transcriptional regulatory domain has been removed; then producing a fragment including a base sequence encoding at least an adenovirus from the vector DNA (1) and a fragment including a base sequence encoding at least E1A, E1B and any extraneous transcriptional regulatory domain from the vector DNA (2'); and thereafter linking the two fragments to make the one vector DNA.

In accordance with the present invention, the production method is performed by preferably using the one DNA strand made by linking the fragment including the base sequence encoding the adenovirus, obtained by cleaving the vector DNA (1) with restriction enzymes I-Ceu I and PI-Sce I, to the fragment including the base sequence encoding any extraneous transcriptional regulatory domain, E1A and E1B, obtained by cleaving the vector DNA (2') with I-Ceu I and PI-Sce I.

For the vector DNA, any transcriptional regulatory domains or any combinations thereof may be used, and furthermore combinations of the E1A and E1B genes and other genes, such as suicide genes and cytokine genes, using an internal ribosome entry site may be also used. In addition, the finally produced chimeric adenovirus may be used when target cells are cells expressing CD46, and is thus particularly useful for cells with low or no CAR expression, compared to conventional type 5 cytolytic viruses.

In a chimeric adenovirus according to the present invention, the obtained chimeric adenovirus becomes a cytolytic virus lysing target cells when using a tissue-specific promoter as an extraneous transcriptional regulatory domain. Also, the obtained chimeric adenovirus becomes an oncolytic virus lysing target tumor cells when using a tumor promoter of, e.g., a midkine, survivin or cyclooxygenase-2 gene as the extraneous transcriptional regulatory domain.

In an adenovirus type 5 according to the present invention, a chimeric adenovirus, in which a fiber knob domain is replaced by an adenovirus type 35 fiber knob domain and any extraneous transcriptional regulatory domain which controls expression of E1A and E1B genes is introduced into a region from which a type 5 E1A transcriptional regulatory domain has been removed, particularly a cytolytic virus which lyses target cells or an oncolytic virus which lyses target tumor cells, may be made to be a medicament without being processed or a medicament containing cells infected with the virus as active ingredients. In addition, the cytolytic or oncolytic virus may be used alone or in enclosed form into a liposome or nanoparticles as a carrier, or a cell itself infected with the virus may be used, as a medicament for killing and damaging only target cells by *in vitro* or *in vivo* administration of it into a cell group, having target cells, or a tissue, or into the vein/artery. In this use, the infection of the cells of interest can be also enhanced by physical or electric action by, e.g., ultrasonic wave or electroporation.

In case of targeting tumor cells in particular, in the majority of human tumors, a chimeric cytolytic virus according to the present invention exhibits efficacy and can be expected to also show a combined effect with another anticancer agent or radiation therapy. Specifically, it is useful for therapies for intractable digestive system tumors such as esophageal, liver, pancreatic, large bowel and gallbladder cancers, and metastatic focuses thereof, or for cancers, for which exogenous administration of the virus is comparatively easily carried out, such as brain tumor, malignant melanoma, head and neck cancer, lung cancer and breast cancer; and tumors that metastasize to the pleura or peritoneum and intrathoracically or intraperitoneally progress, such as malignant mesothelioma, lung cancer and ovarian cancer.

A cytolytic or oncolytic virus according to the present invention may be administered without being processed or with a pharmaceutically acceptable excipient, which is typically used, following its formulation in form of a solution, a suspension, a gel, or the like. It may be administered by, e.g., local injection as a dosage form. Its administration may be also systemic administration such as typical intravenous or intraarterial administration. Its adminisitration may be also carried out by infecting cells with the virus and thereafter administering the infected cells in the above-mentioned forms. Its dose, which depends on the condition, age, sex, route of administration, and dosage form of a subject to be administered, is generally 1 x 10¹⁰ plaque forming unit (pfu) to 3 x 10¹² pfu per dose on the basis of the active principles for adults. The present invention is described in more detail below referring to Examples and Reference Examples but is not limited thereto.

### Examples

### [Reference Example 1]

Taking esophageal cancer and mesothelioma malignant for instance, the expression levels of CAR molecules and CD46 were examined using flow cytometry. An HEK293 cell used in production of an adenovirus was used as a control. The results are shown in Table 1.

**[Table 1]**

| | | |
|---|---|---|
| Cells | CAR expression (%) | CD46 expression (%) |

In Table 1, NCI-H2452, NCI-H2052, NCI-H226, NCI-H28 and MSTO-211H cells are human malignant mesothelioma cells, and the other cells are human esophageal cancer cells. The expression levels of CAR and CD46 of each cell are shown by values based on 100% of a human fetal kidney cell (HEK293 cell). It is clear from Table 1 that the human malignant mesothelioma and esophageal cancer cells have lower CAR molecule expression levels than that of the control HEK263 cell and higher CD46 molecule expression levels than that of the HEK293 cell. Based on such data, for human cells, an adenovirus type 35 with a CD46 molecule as a receptor is estimated to have far higher infection efficiency than that of a type 5 with a CAR molecule as a receptor.

### [Reference Example 2]

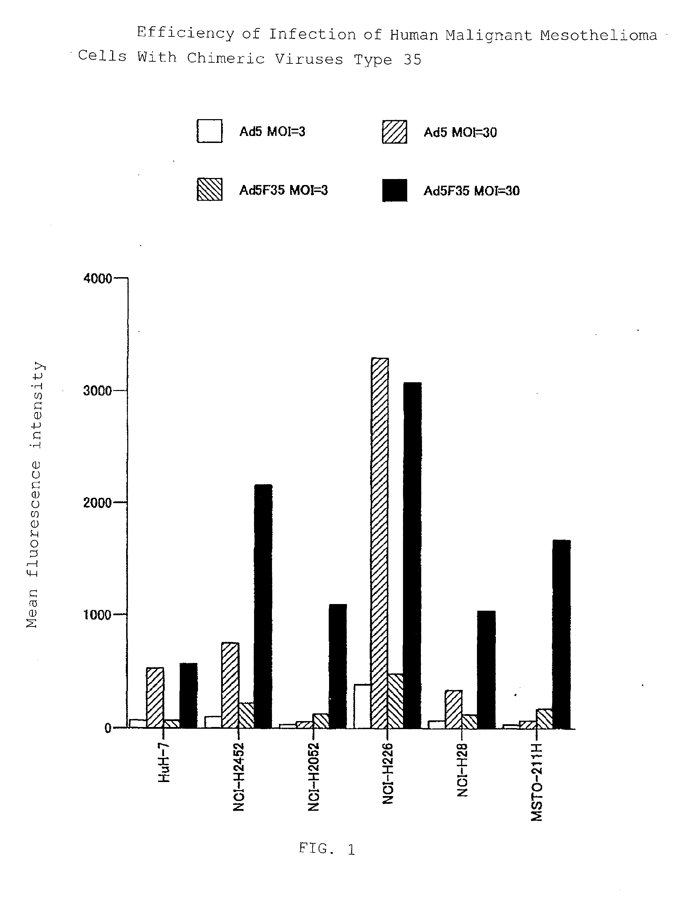

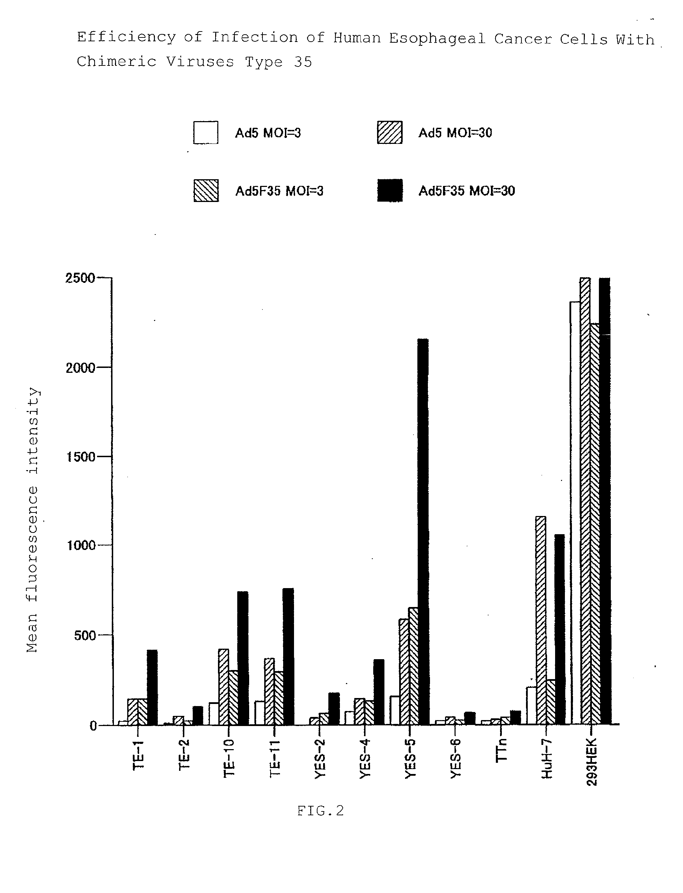

The gene transfer efficiency of a chimeric adenovirus having a type 35 fiber knob domain was examined. The chimeric virus having the type 35 fiber knob domain among chimeric adenovirus vectors commercially available from Avior Therapeutics, Inc. (Seattle, U.S.A.) was used to examine gene transfer efficiency for human malignant mesothelioma and human esophageal cancer (the cells used were a human liver carcinoma cell HuH-7 and the other cells identical to those in Table 1). Vectors which can express GFP with a cytomegalovirus promoter were used to infect the cells with a chimeric virus of type 5 (Ad5-GFP) or type 35 (Ad5F35-GFP) at a fixed MOI for 30 minutes, the cells were washed, and, 2 days thereafter, the GFP expression levels were examined on the basis of mean fluorescence intensity as an index using flow cytometry. The results are shown in FIG. 1 (malignant mesothelioma) and FIG. 2 (esophageal cancer).

As a result, as is clear from FIG. 1, the human liver carcinoma cell HuH-7 used as a control exhibited similar gene expression levels in both Ad5-GFP and Ad5F35-GFP, whereas all the examined human mesothelioma malignant cells exhibited higher gene expression in Ad5F35-GFP; and this means that the type 35 chimeric virus has good infection efficiency compared to the type 5. Ditto with the human esophageal cancer, the results, as shown in FIG. 2, exhibits that the esophageal cancer cells in comparison with the controls, the HuH-7 and HEK293 cells, had better gene expression and infection efficiency in Ad5F35-GFP than those in Ad5-GFP.

### [Example 1]

Conventional production of non-propagative chimeric adenoviruses is a method depending on a homologous gene recombination mechanism in *E. coli* or HEK293 cells, which comprises, in a non-propagative chimeric adenovirus-producing kit from Avior Therapeutics Inc., also inserting a gene of interest into an LHSP vector and simultaneously transfecting it and an RHSP vector having a type 35 fiber knob domain into the HEK293 cells to produce the viruses by homologous gene recombination in the cells. This method was not only extremely non-effective but also had an extremely low frequency of intracellular homologous gene recombination, and required individual examination of whether obtained plaques contained a virus of interest.

Therefore, in the present invention, all obtained plaques were made to contain viruses of interest by transfecting previously-integrated virus DNA into HEK293 cells as well as virus-producing cells by techniques as described below using pShuttle2 and pAdeno-X vectors from Clontech Inc.

### [Production of Vector DNA (1) Facilitating Production of Chimeric Adenovirus]

The adenovirus type 5 fiber knob domain is encoded between a domain, in which E3B 14.7K molecules are encoded, and a domain, in which E4 ORF6/7 molecules are encoded, including a CAR binding domain corresponding to 31042-32787 in Accession Number: M73260. For cleaving this CAR binding domain using restriction enzymes to obtain fragments thereof, Eco RI fragments (23.9-29.4 kb) of pAdeno-X from Clontech Inc. can be easily used, and the fragments contain a type 5 CAR binding domain (corresponding to 24.5-26.2 kb in pAdeno-X). In contrast, a type 35 fiber knob domain corresponds to 30827-33609 in Accession Number: AY271307, including a CD46 binding domain corresponding to 30956-31798. For cleaving the type 35 domain using restriction enzymes to obtain fragments thereof, Eco RI fragments (23.8-29.0 kb) of RHSP Ad35 from Avior Therapeutics Inc. can be easily used, and the fragments contain a type 35 CD46 binding domain (corresponding to 24.8-25.8 kb in RHSP Ad35). Therefore, DNA, in which an Eco RI fragment corresponding to 23.9-29.4 kb was removed from pAdeno-X, and an Eco RI fragment corresponding to 23. 8-29. 0 kb, which was cleaved from RHSP Ad35, were bound to finish pAd5F35 (SEQ ID NO. 1) (vector DNA (1)).

### [Production of Vector DNA (2) Facilitating Production of Chimeric Adenovirus]

In order to first produce a vector having a multicloning site, pShuttle2 from Clontech Inc. was cleaved with Mun I and Nhe I to remove a cytomegalovirus promoter domain (corresponding to 184-918 of pShuttle2), and DNA having a multicloning site of Mun I-Sca I-Bam HI-Eco RV-Sal I was bound to this region to finish pS-PL. As a result, a cloning site of Mun I-Sca I-Bam HI-Eco RV-Sal I-Nhe I-Dra I-Apa I-Xba I-Not I-Bst XI-Kpn I-Aff II was present in the pS-PL.

The domains of adenovirus type 5 E1A and E1B genes correspond to 560-3509 in Accession Number: M73260, and a transcriptional regulatory domain in the domain corresponds to 341-548 in Accession Number: M73260. Therefore, vector DNA (2) which controls expression of the E1A and E1B genes was produced by a method as described below based on an extraneous transcriptional regulatory domain introduced into a region from which the transcriptional regulatory domain of the E1A and E1B genes was removed.

LHSP of 4276 bp, in which LHSP from Avior Therapeutics Inc. was cleaved with Xba I and Mun I to remove a DNA fragment of 438 bp, and a DNA fragment (2585 bp) including a part of E1A and E1B genes, obtained by cleaving pXC1 (Microbix Biosystems Inc., Ontario, Canada) with Xba I and Mun I, were bound. The DNA produced thereby is defective in the transcriptional regulatory domain of E1A and E1B genes in adenovirus sequences, includes a multicloning site as a substitute therefor, and has 22-341 and 1338-5784 in Accession Number: M73260. A sequence corresponding to 549-1344 (with Xba I site present in a 3' region) in M73260 was then amplified using primer designed to have a Bam HI in a 5' region and Xba I in a 3' region using PCR. In addition, the PCR products were cleaved with Bam HI and Xba I, followed by inserting the cleaved products into the Bam HI site of the multicloning site of the DNA and into the Xba I site of the E1A domain.

The DNA competed thereby has 22-341 and 549-5784 in Accession Number: M73260 and includes Cla I and Bam HI sites present in a region defective in 342-548. A domain including E1A and E1B genes (549-3923 in M73260), in which this DNA was cleaved with Cla I and Mun I, was inserted into the Not I site of the pS-PL to produce pS-PL/E1A-E1B (SEQ ID NO. 2). Finally finished pS-PL/E1A-E1B is a vector (vector DNA (2)) which can control expression of E1A and E1B genes under control of any transcriptional regulatory domains inserted into a plurality of cloning sites.

### [Example 2]

### [Production of Cytolytic Chimeric Viruses by Promoters of Midkine, Survivin, and Cyclooxygenase-2 (COX-2) Genes]

The transcriptional regulatory domains of midkine, survivin and COX-2 genes exhibiting high expression in tumors were cloned using PCR. The domains of from -559 to +50 for the midkine, of from -478 to +43 for the survivin, and of from -327 to +59 for the COX-2, when each transcription start site was indicated by +1, were inserted into the Eco RV sites of the pS-PL/E1A-E1B to obtain plasmid DNA. In addition, as a control, cytomegalovirus promoters were inserted into identical sites. In addition, as a control for a replication-competent virus, in order to produce a non-propagative virus, LacZ cDNA cleaved from pCH110 from Pharmacia Corporation with Hind III and Bam HI was inserted into pShuttle2 (Clontech Inc.).

The aforementioned plasmid DNA was cleaved with I-Ceu I and PI-Sce I, these fragments were linked to the fragments obtained by cleaving pAd5F35 with I-Ceu I and PI-Sce I to produce chimeric adenoviruses, and undigested pAd5F35 was further cleaved with Swa I. In addition, as a control, the pS-PL/E1A-E1B including the aforementioned midkine, survivin and COX-2 genes and cytomegalovirus promoters was cleaved with I-Ceu I and PI-Sce I to produce type 5 viruses, and these fragments were linked to the fragments obtained by cleaving pAdeno-X from Clontech Inc. with I-Ceu I and PI-Sce I. In addition, undigested pAdeno-X was similarly cleaved with Swa I.

Each DNA obtained as described above was transformed into *E. coli* DH5α. From the *E. coli,* the *E. coli* having the concerned transcriptional regulatory domains present in the 5' upstreams of the E1A and E1B genes and the plasmid DNAs having the adenovirus DNAs (both chimeric and type 5) except the E3 domain was selected, and the concerned DNAs were extracted from the selected *E. coli.*

The DNAs obtained in the aforementioned extraction were cleaved with Pac I, followed by transfecting the cleaved DNAs into HEK293 cells to perform formation of plaques by CPE. The cultured HEK293 cells and the culture solution thereof were collected, repeatedly freeze-thawed, and thereafter centrifuged at 3,000 rpm for 10 minutes using a centrifuge for cell separation to preserve a supernatant thereof as a first virus solution. The virus solution was used to infect the HEK293 cells with viruses to make second and third virus solutions by similar treatment. The concerned virus was purified from 100 ml of the third virus solution using an adenovirus purification kit from Clontech Inc. Thus, Ad5F35/MK, Ad5F35/Sur, Ad5F35/COX-2 and Ad5F35/CMV having the midkine, survivin and COX-2 genes and the cytomegalovirus promoter as cytolytic chimeric viruses, Ad5/MK, Ad5/Sur, Ad5/COX-2 and Ad5/CMV having similar transcriptional regulatory domains as cytolytic viruses type 5, and Ad5F35/LacZ and Ad5/LacZ as a non-propagative chimeric virus and a virus type 5 were purified by the above-mentioned method. These viruses were confirmed to be viruses of interest, respectively, and mixed with no other viruses by PCR using appropriate primers. In addition, the absorbances of the purified viruses were measured at 260 nm to calculate viral loads according to OPU/ml (optical particle unit) = OD260 x viral dilution x 1.1 x 10¹².

In addition, in HEK293 cells, E1A genes are expressed, and wild-type viruses can intracellularly occur due to gene recombination in theory. Therefore, when producing the aforementioned cytolytic viruses, whether the production of the viruses was possible or not was examined by a similar technique using HuH-7 cells including no adenovirus genes. As a result, it was confirmed by similar PCR that, even if using cells including no adenovirus genes such as HuH-7 cells, cytolytic viruses can be produced, and in this case, unsurprisingly, viruses with different structures such as wild-type viruses do not occur.

Whether actual infection with the chimeric viruses according to the present invention occurred in CD46 as a receptor was examined using Ad5F35/LacZ and Ad5/LacZ. Since no mouse cells are infected with adenoviruses type 35, human CD46 molecules were expressed in mouse malignant melanoma B16 cells to produce B16/CD46 cells. The B16 cells were not infected with Ad5F35/LacZ while they were infected with Ad5/LacZ under a high MOI, whereas the B16/CD46 cells were infected with Ad5F35/LacZ and also infected with Ad5/LacZ under a high MOI. Specifically, these results exhibit that the viruses produced by the aforementioned method were chimeric viruses type 5 as expected.

### [Example 3]

### [Anti-tumor Activity (1) of Chimeric Cytolytic Virus of the Present Invention]

In order to examine the cytotoxic activity of each virus produced, taking mesothelioma malignant for instance, 1 x 10⁴ target cells were inoculated on a 96-well plate, it was infected with viruses at fixed OPU/ml, and minimum OPU/ml with occurrence of 50% or higher of CPE was calculated. The results are shown in Table 2.

**[Table 2]**

| |
|---|
| Cells |
| Viruses |

In Table 2, H2452^{a} is NCI-H2452, H2052^{b} is NCI-H2052, H226^{c} is NCI-H226, H28^{d} is NCI-H28, and 211H^{e} is MSTO-211H.

The results in Table 2 exhibits the cytotoxic activities in case of using the fixed viral load, in which a lower value shows higher sensitivity of the cell to the virus. In addition, Ad5/LacZ and Ad5F35/LacZ have no cytotoxic activities because of being non-propagative. Although comparison between cells is impossible because there are many factors to determine virus sensitivity in each cell and the sensitivity depends on each cell, comparisons between the cytotoxic activities of chimeric adenoviruses according to the present invention and adenoviruses type 5 having identical transcriptional regulatory domains show that the chimeric adenoviruses of the present invention have at least similar cytotoxic activity to the type 5 in all the cells, and, in particular, the cytotoxic activities of the chimeric adenoviruses are higher than those of the type 5 in the cells with the decreased CAR molecule expression (NCI-H2052 and MSTO-211H).

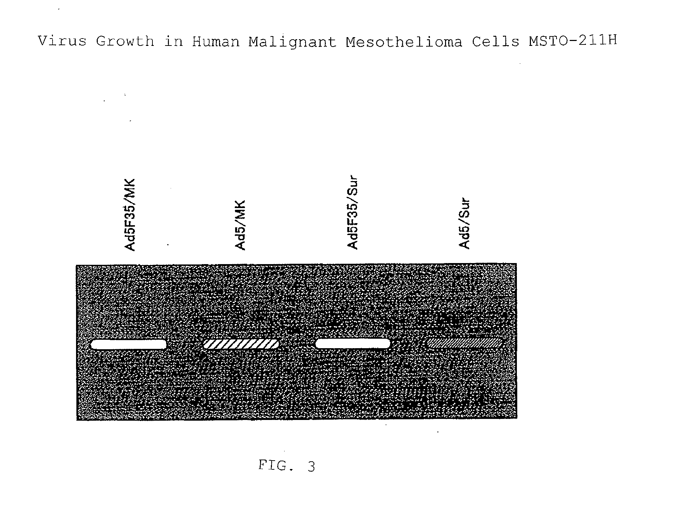

In the aforementioned cells, actual viral growth was examined using PCR. The human malignant mesothelioma MSTO-211H cells were infected with Ad5/MK, Ad5F35/MK, Ad5/Sur and Ad5F35/Sur at the fixed MOI, their culture supernatants were collected following occurrence of CPE, and DNAs were extracted from the supernatants. The DNAs and primers directed at both E1B and adenovirus vector DNA were used to carry out PCR for 20 cycles. From FIG. 3, in which the results were shown, the number of viruses produced in the infection with Ad5F35/MK or Ad5F35/Sur was found to be larger than that in the infection with Ad5/MK or Ad5/Sur. Because the chimeric adenovirus according to the present invention differs from the virus type 5 only in a fiber knob domain, the difference of production of the viruses reflect that of their infection efficiencies, and the chimeric adenovirus of the present invention was thus found to more efficiently effect cytolysis than the virus type 5.

### [Example 4]

### [Anti-tumor Activity (2) of Chimeric Cytolytic Virus of the Present Invention]

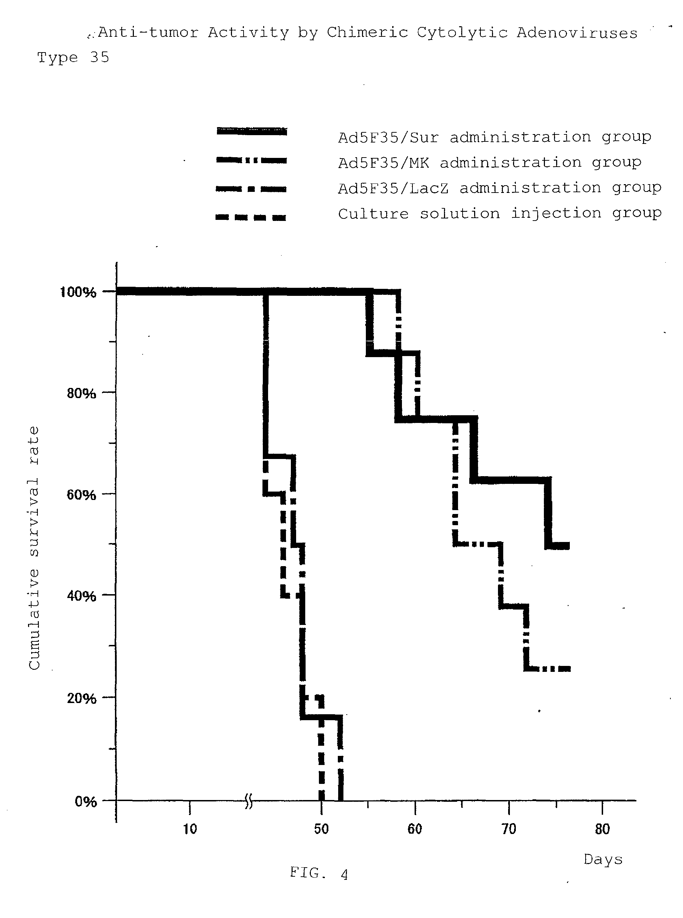

To verify the anti-tumor activity of the chimeric adenoviruses according to the present invention *in vivo,* nude mice (six or seven per group) were intraperitoneally inoculated with 3 x 10⁶ MSTO-211H cells and were intraperitoneally administered with Ad5F35/MK, Ad5F35/Sur and Ad5F35/LacZ at 2 x 10⁸ pfu and each 300 µl of culture solution as a control at 4, 5, 6, 9, 10 and 11 days after the inoculation, and subsequent cumulative survival rates were examined by Kaplan-Meier method. The results are shown in FIG. 4. As can be seen from FIG. 4, the survival time in the group administered with Ad5F35/MK or Ad5F35/Sur was significantly extended (P < 0.001) in comparison with the control group Ad5F35/LacZ or the culture solution-inoculated group. In other words, the chimeric cytolytic viruses according to the present invention were found to be able to exhibit an efficacious therapeutic effect on malignant mesothelioma for which no efficacious therapeutic methods had been available.

### Industrial Applicability

Chimeric adenoviruses according to the present invention have high gene transfer efficiency because of having a type 35 fiber knob domain, also have a strong cytotoxicity reaction on a number of human target cells compared to a conventional cytolytic adenovirus type 5 because of being integrated with a transcriptional regulatory domain specific to expression in target cells, and may be efficacious pharmaceutical preparations for damaging tumors in particular. Accordingly, for example, a chimeric cytolytic adenovirus according to the present invention is an excellent anticancer agent, which can be used in combination with radiotherapy or chemotherapy because it differs from radiation therapy or conventional anticancer agents in the mechanism of action. Also, cytolytic adenoviruses according to the present invention may be utilized for damaging cells/tissues of interest *in vitro* or *in vivo*. In addition, such chimeric adenoviruses can be easily produced using a particular vector system by a method according to the present invention.

## Claims

1. A chimeric adenovirus, in which a fiber knob domain in an adenovirus type 5 is replaced by an adenovirus type 35 fiber knob domain and any extraneous transcriptional regulatory domain controlling expression of E1A and E1B genes is introduced into a region from which a type 5 E1A transcriptional regulatory domain has been removed.

2. The chimeric adenovirus according to claim 1, wherein the extraneous transcriptional regulatory domain is a tissue-specific promoter.

3. The chimeric adenovirus according to claim 1, wherein the extraneous transcriptional regulatory domain is a tumor promoter.

4. The chimeric adenovirus according to claim 3, wherein the tumor promoter is a promoter of a midkine, survivin or cyclooxygenase-2 gene.

5. The chimeric adenovirus according to claim 1, comprising a base sequence provided by introducing a base sequence encoding any extraneous transcriptional regulatory domain into a base sequence as shown in SEQ ID NO. 3 in the sequence listing or a base sequence homologous thereto.

6. A method of producing a chimeric adenovirus, comprising using one vector DNA made by linking a fragment including a base sequence encoding an adenovirus obtained by vector DNA (1), in which an adenovirus type 5 fiber knob domain is replaced by an adenovirus type 35 fiber knob domain, to a fragment including: any extraneous transcriptional regulatory domain provided by produced vector DNA (2'), in which any extraneous transcriptional regulatory domain is introduced into vector DNA (2) for use in introduction of any extraneous transcriptional regulatory domain that controls expression of adenovirus type 5 genes E1A and E1B into a region from which an adenovirus type 5 E1A transcriptional regulatory domain has been removed; and a base sequence encoding E1A and E1B, when producing the chimeric adenovirus according to claim 1.

7. The method of producing the chimeric adenovirus according to claim 6, wherein the vector DNA (1) is vector DNA (pAd5F35) having a base sequence as shown in SEQ ID NO. 1 in the sequence listing or a base sequence homologous thereto.

8. The method of producing the chimeric adenovirus according to claim 6 or 7, wherein the vector DNA (2) is vector DNA (pS-PL/E1A-E1B) having a base sequence as shown in SEQ ID NO. 2 in the sequence listing or a base sequence homologous thereto.

9. A medicament comprising: a chimeric adenovirus, in which a fiber knob domain in an adenovirus type 5 is replaced by an adenovirus type 35 fiber knob domain and any extraneous transcriptional regulatory domain controlling expression of E1A and E1B genes is introduced into a region from which a type 5 E1A transcriptional regulatory domain has been removed; or a cell infected with the virus.

10. The medicament according to claim 9, wherein the chimeric adenovirus is a cytolytic virus that lyses a target cell or an oncolytic virus that lyses a target tumor cell.
